# EUROPEAN PATENT SPECIFICATION

(11) **EP 2 327 987 B1**
(45) Date of publication and mention of the grant of the patent: **21.08.2013**
(21) Application number: 10193078.2
(22) Date of filing: 30.11.2010
(51) Int. Cl.: G01N 33/576

(54) **Method and kit for detection of HCV core protein**
Verfahren und Reagenzkit zur Bestimmung von HCV-Kernprotein
Procédé et kit de réactif pour la détection de la protéine centrale HCV

(30) Priority: 30.11.2009 JP 2009272339; 28.12.2009 JP 2009298581; 26.02.2010 JP 2010042217
(43) Date of publication of application: 01.06.2011
(73) Proprietor: SYSMEX CORPORATION, Kobe-shi, Hyogo 651-0073 (JP)
(72) Inventor: Yamagaito, Takahiro, Kobe-shi, Hyogo 651-0073 (JP); Takeda, Kazuhiko, Kobe-shi, Hyogo 651-0073 (JP); Koma, Takuya, Kobe-shi, Hyogo 651-0073 (JP)
(74) Representative: Paemen, Liesbet R.J.

(56) References cited:
- EP-A1- 0 967 484
- JP-A- 58 052 564
- JP-A- 2001 004 621
- US-A- 5 064 768
- US-A- 5 236 849
- US-A- 5 714 371
- US-A- 5 775 342
- US-A- 5 989 806
- US-A- 5 990 298
- US-A1- 2004 137 588
- US-A1- 2008 044 807
- KASHIWAKUMA T ET AL: "Detection of hepatitis C virus specific core protein in serum of patients by a sensitive fluorescence enzyme immunoassay (FEIA)", JOURNAL OF IMMUNOLOGICAL METHODS, ELSEVIER SCIENCE PUBLISHERS B.V.,AMSTERDAM, NL, vol. 190, no. 1, 28 March 1996 (1996-03-28), pages 79-89, XP004020891, ISSN: 0022-1759, DOI: DOI:10.1016/0022-1759(95)00261-8
- MOROTA K ET AL: "A new sensitive and automated chemiluminescent microparticle immunoassay for quantitative determination of hepatitis C virus core antigen", JOURNAL OF VIROLOGICAL METHODS, ELSEVIER BV, NL, vol. 157, no. 1, 1 April 2009 (2009-04-01) , pages 8-14, XP025994039, ISSN: 0166-0934, DOI: DOI:10.1016/J.JVIROMET.2008.12.009 [retrieved on 2009-01-20]
- TOKITA HAJIME ET AL: "Hepatitis C virus core mutations reduce the sensitivity of a fluorescence enzyme immunoassay", JOURNAL OF CLINICAL MICROBIOLOGY, vol. 38, no. 9, September 2000 (2000-09), pages 3450-3452, XP002640230, ISSN: 0095-1137

### SEQUENCE LISTING

<110> Sysmex corporation
<120> METHOD FOR PRETREATING SAMPLE FOR DETECTION OF HCV CORE PROTEIN, REAGENT KIT FOR DETECTION OF HCV CORE PROTEIN, METHOD FOR DETERMINING THE PRESENCE OR ABSENCE OF HEPATITIS C VIRUS IN SAMPLE, AND METHOD FOR IMMUNOASSAY OF HCV
<130> SYSM-045-EP
<160> 8
<170> PatentIn version 3.1
<210> 1
   <211> 160
   <212> PRT
   <213> Hepatitis C virus
<400> 1
   Met Ser Thr Asn Pro Lys Pro Gln Arg Lys Thr Lys Arg Asn Ala Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Asp Arg Arg Ser Thr Gly Lys Ser Trp Gly Lys Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Thr Trp Gly Leu Thr Asp Pro
               100                 105                 110
   Arg His Arg Ser Arg Asn Leu Gly Lys Val Ile Asp Thr Ile Thr Cys
           115                 120                 125
   Gly Phe Ala Asp Leu Met Gly Tyr Ile Pro Val Val Gly Ala Pro Val
       130                 135                 140
   Gly Gly Val Ala Arg Ala Leu Ala His Gly Val Arg Val Leu Glu Asp
   145                 150                 155                 160
<210> 2
   <211> 160
   <212> PRT
   <213> Hepatitis C virus
<400> 2
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Thr Trp Ala Gln Pro Gly
   65 70 75 80
   Tyr Pro Trp Pro Leu Tyr Gly Asn Glu Gly Met Gly Trp Ala Gly Trp
   85 90 95
   Leu Leu Ser Pro Arg Gly Ser Arg Pro Ser Trp Gly Pro Thr Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Leu
   130 135 140
   Gly Gly Ala Ala Arg Ala Leu Ala His Gly Val Arg Val Leu Glu Asp
   145 150 155 160
<210> 3
   <211> 160
   <212> PRT
   <213> Hepatitis C virus
<400> 3
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Pro Arg Lys Thr Ser Glu Arg Ser Gln Pro Arg Gly Arg Arg Gln Pro
   50 55 60
   Ile Pro Lys Ala Arg Arg Pro Glu Gly Arg Thr Trp Ala Gln Pro Gly
   65 70 75 80
   Tyr Pro Trp Pro Leu Tyr Gly Asn Glu Gly Met Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
<210> 4
   <211> 160
   <212> PRT
   <213> Hepatitis C virus
<400> 4
   Met Ser Thr Leu Pro Lys Pro Gln Arg Gln Thr Lys Arg Asn Thr Tyr
   1               5                   10                  15
   Arg Arg Pro Gln Asn Val Lys Phe Pro Gly Gly Gly Gln Ile Val Gly
               20                  25                  30
   Gly Val Tyr Val Leu Pro Arg Arg Gly Pro Arg Leu Gly Val Arg Ala
           35                  40                  45
   Val Arg Lys Thr Ser Glu Arg Ser Gln Pro Arg Gly Arg Arg Gln Pro
       50                  55                  60
   Ile Pro Lys Ala Arg Ser Arg Glu Gly Arg Ser Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Asn Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Ile Gly Ala Pro Val
       130                 135                 140
   Gly Gly Val Ala Arg Ala Leu Ala His Gly Val Arg Ala Leu Glu Asp
   145                 150                 155                 160
<210> 5
   <211> 20
   <212> PRT
   <213> Hepatitis C virus
<400> 5
   Asp Val Lys Phe Pro Gly Gly Gly Gln Ile Val Gly Gly Val Tyr Leu
   1               5                   10                  15
   Leu Pro Arg Arg
               20
<210> 6
   <211> 10
   <212> PRT
   <213> Hepatitis C virus
<400> 6
   Asp Val Lys Phe Pro Gly Gly Gly Gln Ile
   1               5                   10
<210> 7
   <211> 20
   <212> PRT
   <213> Hepatitis C virus
<400> 7
   Gly Pro Arg Leu Gly Val Arg Ala Thr Arg Lys Thr Ser Lys Arg Ser
   1               5                   10                  15
   Gln Pro Arg Gly
               20
<210> 8
   <211> 10
   <212> PRT
   <213> Hepatitis C virus
<400> 8
   Gly Pro Arg Leu Gly Val Arg Ala Thr Arg
   1               5                   10

## Description

### TECHNICAL FIELD

The invention relates to a method for detection of an HCV core protein, and a reagent kit for detection of an HCV core protein.

### BACKGROUND ART

Hepatitis C is an infectious disease caused by the hepatitis C virus (HCV). There is no established prophylaxis for hepatitis C, and it is considered that most infected individuals (carriers) can develop cirrhosis or liver cancer in 10 to 30 years. Therefore, early detection and early treatment of HCV infection are very important.

HCV, which causes hepatitis C, is an enveloped RNA virus with a diameter of about 60 nm. The HCV RNA is a positive single strand of about 9,500 nucleotides. Its gene structure, which resembles that of flavivirus, has untranslated regions (UTRs) at the 5' and 3' ends and a translated region encoding about 3,000 amino acids between the UTRs. The translated region has a core region encoding the structural protein, envelope region 1, envelope region 2, and a nonstructural region following envelope region 2, which are located in this order from the 5' side.

In a known method for detecting HCV, the HCV core protein encoded by the core region is detected as an antigen using an immunological technique. The HCV core protein exists inside the enveloped HCV. Therefore, the HCV core protein has to be separated from HCV so that the HCV core protein can be detected as an antigen. A known pretreatment method for separating the HCV core protein from HCV includes treating HCV with an alkaline solution and then neutralizing it with an acid solution. For example, Japanese Patent Application Laid-Open No. 2001-004621 discloses a pretreatment method in which the HCV core protein is separated by a process including: a first treatment step of treating a sample suspected of containing hepatitis C virus with a reagent containing an nonionic surfactant, a protein denaturing agent, and an alkaline material; and a second treatment step of treating the sample obtained in the first treatment step with an acid material-containing reagent.

A known HCV test is performed by an antibody assay in which the HCV core protein encoded by the core region is used as an antigen. However, the HCV test by the antibody assay cannot detect hepatitis C until antibodies are produced after the HCV infection. Therefore, early detection of HCV is difficult. The antibody test also has a problem in which in principle, it cannot determine whether the subject is a cured patient after the infection or an actively infected patient.

An HCV test to solve such problems includes a method of directly detecting an HCV antigen. Concerning such a method, a known method includes detecting an HCV core antigen in blood serum using a monoclonal antibody specific to the HCV core antigen (US7776542).

However, there has been a demand for an HCV detection method having higher sensitivity than the above measurement method, because the amount of viral particles in an HCV-infected patient is very small.

When the presence or absence of HCV infection is determined, it is necessary to detect a very small amount of HCV in a biological sample collected from the subject. Therefore, high sensitivity is required for the detection of HCV. In this regard, an immune complex transfer assay is known as a method for detecting an antigen with high sensitivity (US5236849). The immune complex transfer assay is characterized by including transferring, between two or more solid phases, an immune complex containing the substance to be measured and then measuring the immune complex on the solid phase. When the immune complex containing the substance to be measured is transferred between two or more solid phases, contaminants are removed, so that nonspecific signals can be reduced in the detection system. Therefore, the use of the immune complex transfer assay makes it possible to detect the target substance with high sensitivity.

However, HCV, which causes hepatitis C, is an RNA virus known to undergo a high rate of mutation. For example, when the 49th amino acid of the HCV genotype 1b core region protein set forth in SEQ ID NO:2 is mutated, HCV sometimes cannot be detected with high sensitivity even by the immune complex transfer assay with a known antibody (Journal of Clinical Microbiology, Sept. 2000, pp. 3450-3452).

### SUMMARY OF THE INVENTION

A sample for detection of the HCV core protein can be simply prepared in a short time using the method disclosed in Japanese Patent Application Laid-Open No. 2001-004621, which includes treating HCV with an alkaline solution and then neutralizing it with an acid solution. However, when the sample prepared by such a method is subjected to detection of the HCV core protein by an immunoassay using particles, the particles dispersed in a liquid phase can aggregate after B/F separation.

In addition, when the sample prepared by such a method is subjected to detection of the HCV core protein by an immune complex transfer assay, the HCV core protein sometimes cannot be sufficiently detected. As a result of a further investigation, it has been found that when the HCV core protein contained in the sample prepared by the method disclosed in Japanese Patent Application Laid-Open No. 2001-004621 is detected by an immune complex transfer assay using particles as a solid phase, the particles dispersed in a liquid phase sometimes aggregate after B/F separation is performed. It is considered that the particle aggregation can cause the problem of a reduction in detection sensitivity in an immune complex transfer assay using particles.

An object of the invention is to provide an immune complex transfer assay capable of detecting HCV with high sensitivity and to provide a reagent kit for use therein. More particularly, provided herein are methods for detecting a hepatitis C virus (HCV) core protein in a sample by an immune complex transfer assay using particles as a solid phase, comprising: treating the sample suspected of containing hepatitis C virus with an alkaline material-containing reagent, which contains an alkaline material, a nonionic surfactant and a chaotropic agent; treating the sample obtained in the first treating step with an acid material-containing reagent which contains an acid material and a reducing agent, wherein the reducing agent is at least one selected from mercaptoethylamine, mercaptoethanol, dithiothreitol, cysteine, dithioerythritol, sodium borohydride, and phosphine, and detecting said HCV core protein in said sample obtained in the second step with an immune complex transfer assay using particles as a solid phase.

According to particular embodiments of the present invention, the nonionic surfactant may be selected from polyoxyethylene nonionic surfactants; the chaotropic agent may be at least one selected from urea, guanidine hydrochloride, sodium salicylate, acetamide, and formaldehyde; the alkaline material may be at least one selected from sodium hydroxide, potassium hydroxide, and magnesium hydroxide; the acid material may be at least one selected from acetic acid, lactic acid, citric acid, malic acid, succinic acid, phosphoric acid, formic acid, fumaric acid, tartaric acid, hydrochloric acid, and sulfuric acid; the particles may be magnetic particles, and, at least one of the reagents used in the first and second treating steps may contain an inorganic salt. According to the invention, the inorganic salt may be at least one selected from sodium chloride, potassium chloride, magnesium chloride, and sodium sulfate. When using magnetic particles, the immune complex transfer assay may comprise forming a complex comprising a HCV core protein, an anti-HCV core protein antibody, and magnetic particles in a liquid phase, separating the complex from the liquid phase by magnetic separation, and detecting the HCV core protein.

According to particular embodiments of the present invention, the immune complex transfer assay using particles as a solid phase may comprise: forming a complex comprising a labeled antibody, a first antibody binding to a HCV core protein and the HCV core protein contained in the sample obtained in the second treating step on particles, the labeled antibody is capable of binding to the HCV core protein; separating the particles in the sample from other components in the sample after the forming step; transferring the complex formed on the particles obtained in the separating step to a solid phase other than the particles; measuring the label of the complex obtained in the transferring step and transferred to the solid phase other than the particles; and determining whether hepatitis C virus is present or absent in the sample, based on the result of the measuring step. Alternatively, the immune complex transfer assay using particles as a solid phase may comprise: forming, on the particles, a complex comprising a HCV core protein, a labeled antibody binding to the HCV core protein, and a first antibody binding to the HCV core protein; and measuring the label of the complex. In said latter methods the labeled antibody and the first antibody may bind to different sites of the HCV core protein, and the sites to which they bind do not contain the 49th amino acid of the HCV core protein set forth in SEQ ID NO:2. According to the present invention, the site to which the labeled antibody or the first antibody binds may be the amino acid sequence of amino acids 21 to 30 or amino acids 41 to 48 of the HCV core protein set forth in SEQ ID NO:2.

A further aspect of the invention is the provision of a reagent kit for detection of a hepatitis C virus (HCV) core protein by an immune complex transfer assay using particles as a solid phase, comprising: a first reagent containing an alkaline material, a nonionic surfactant and a chaotropic agent; a second reagent containing an acid material and a reducing agent, wherein said reducing agent is at least one selected from mercaptoethylamine, mercaptoethanol, dithiothreitol, cysteine, dithioerythritol, sodium borohydride, and phosphine; a third reagent containing a labeled antibody capable of binding to the HCV core protein; a fourth reagent containing a first antibody capable of binding to the HCV core protein; a fifth reagent containing particles; a sixth reagent containing a second antibody capable of binding to the first antibody and the particles; and a solid phase on which a material capable of binding to the first antibody is immobilized.

According to particular embodiments of the present invention, at least one of the first and second reagents may contain an inorganic salt. In addition, according to particular embodiments of the present invention the labeled antibody and the first antibody may bind to different sites of the HCV core protein, and the sites to which they bind do not contain the 49th amino acid of the HCV core protein set forth in SEQ ID NO:2. According to particular embodiments the present invention, the site to which the labeled antibody or the first antibody binds may be the amino acid sequence of amino acids 21 to 30 or amino acids 41 to 48 of the HCV core protein set forth in SEQ ID NO:2.

The first aspect of the present invention makes it possible to detect HCV core protein with high sensitivity in a sample by an immune complex transfer assay using particles as a solid phase.
The further aspect of the present invention provides a reagent kit for detection of a HCV core protein by an immune complex transfer assay using particles as a solid phase.

### BRIEF DESCRIPTION OF THE DRAWINGS

Fig. 1 is a diagram showing a process to the transferring step in an embodiment of the invention;
Fig. 2 is a graph showing the reactivity of anti-HCV core monoclonal antibodies (HCF4-801 and HE25) to HCV core proteins with the 49th amino acid of SEQ ID NO:2 mutated (HCV-J1bT49P and HCV-3bNE137); and
Fig. 3 is a graph showing the relationship between HCV-RNA quantity and luminescence intensity with respect to each HCV genotype.

The specification describes a method for pretreating a sample for detection of an HCV core protein is a method for pretreating a sample for use in detection of an HCV core protein by an immunoassay using particles, which includes: a first treatment step of treating a sample suspected of containing hepatitis C virus with an alkaline material-containing reagent; and a second treatment step of treating the sample obtained in the first treatment step with an acid material-containing reagent, wherein at least the reagent used in the second treatment step contains a reducing agent.

The reducing agent contained in at least the second treatment steps can inhibit particle aggregation in the separating step described below. The "reducing agent" is at least one selected from mercaptoethylamine, mercaptoethanol, dithiothreitol, cysteine, dithioerythritol, sodium borohydride, or phosphine. As described herein, particularly preferred reducing agents are mercaptoethylamine, dithiothreitol, and cysteine hydrochloride.

The concentration of the reducing agent in at least one of the reagents used in the second treatment step is not restricted and may be appropriately controlled depending on the type of the reducing agent used. For example, when mercaptoethylamine is used as the reducing agent, the concentration of mercaptoethylamine in at least one of the reagents used in the first and second treatment steps is preferably from 10 to 60 mM.

As described herein, at least one of the reagents used in the first and second treatment steps preferably further contains an inorganic salt. The inorganic salt contained in at least one of the reagents used in the first and second treatment steps can further inhibit particle aggregation in the separating step described below. The "inorganic salt" may be any salt having no effect on the forming step described below. As described herein, the salt is particularly preferably an inorganic salt. More specifically, for example, the inorganic salt may be sodium chloride, potassium chloride, magnesium chloride, or sodium sulfate. As described herein, preferred salts are sodium chloride, potassium chloride, and sodium sulfate.

The concentration of the inorganic salt in at least one of the reagents used in the first and second treatment steps is not restricted and may be appropriately controlled depending on the type of the inorganic salt used. For example, when sodium chloride is used as the inorganic salt, the concentration of sodium chloride in at least one of the reagents used in the first and second treatment steps is preferably from 0.1 to 1.0 M.

The "sample suspected of containing HCV" is preferably a biological sample or a sample prepared from a biological sample. Examples of a biological sample and a sample prepared from a biological sample include urine, feces, whole blood, blood plasma, blood serum, bile, gastrointestinal secretions, lymph fluid, semen, bone marrow fluid, saliva, breast milk, tissue extracts, tissue homogenates, cell extracts, and cell homogenates.

As described herein, the reagent used in the first treatment step further contains a nonionic surfactant. The "nonionic surfactant" may be any nonionic surfactant that can be used in the process of dissociating the HCV core protein from HCV. As described herein, the nonionic surfactant is preferably a polyoxyethylene nonionic surfactant. Specific examples of the polyoxyethylene nonionic surfactant include polyoxyethylene alkyl phenyl ethers (such as Triton X-100, Triton X-114, and NP-40), polyoxyethylene sorbitan fatty acid esters (such as Tween-20 and Tween-80), and polyoxyethylene alkyl ethers (such as Brij 35 and Brij 45). As described herein, the polyoxyethylene nonionic surfactant is preferably polyoxyethylene alkyl ether.

The concentration of the nonionic surfactant in the first treatment step is not restricted and may be appropriately controlled depending on the type of the nonionic surfactant used. For example, when polyoxyethylene alkyl ether is used as the nonionic surfactant, the concentration of polyoxyethylene alkyl ether in the first treatment step is preferably from 2 to 4%.

As described herein, the reagent used in the first treatment step further contains a chaotropic agent. The "chaotropic agent" is a material having the property of destabilizing the molecular structure of proteins. Examples of the chaotropic agent include urea, guanidine hydrochloride, sodium salicylate, sodium thiocyanate, sodium perchlorate, acetamide, and formaldehyde. As described herein the chaotropic agent is preferably urea.

The concentration of the chaotropic agent in the first treatment step is not restricted and may be appropriately controlled depending on the type of the chaotropic agent used. For example, when urea is used as the chaotropic agent, the concentration of urea in the first treatment step is preferably from 2 to 4 M.

The "alkaline material" may be any alkaline material that can be used in the process of dissociating the HCV core protein from HCV. Examples of the alkaline material include sodium hydroxide, potassium hydroxide, magnesium hydroxide, and sodium sulfate. As described herein, the alkaline material is preferably sodium hydroxide.

The concentration of the alkaline material in the first treatment step is not restricted and may be appropriately controlled depending on the type of the alkaline material used. For example when sodium hydroxide is used as the alkaline material, the concentration of sodium hydroxide in the first treatment step is preferably from 0.15 to 0.5 N.

The "first treatment step" may be any treatment that includes mixing a sample suspected of containing HCV with an alkaline material-containing reagent as described herein. The first treatment step makes it possible to dissociate the HCV core protein from HCV. The treatment time or the treatment temperature in the first treatment step is not particularly restricted and may be appropriately determined depending on the conditions for the mixing of the sample and the reagent. Specifically, for example, the first treatment step may be performed at a treatment temperature of 20 to 30°C for a treatment time of 6 to 10 minutes, when the reagent used in the first treatment step has a polyoxyethylene nonionic surfactant concentration of 2 to 4%, a urea concentration of 2 to 4 M, and a sodium hydroxide concentration of 0.1 to 0.4 N.

As described herein, examples of the alkaline material-containing reagent include a reagent containing the nonionic surfactant and the chaotropic agent and a reagent kit including two or more reagents each containing each component. For example, the reagent kit may include as alkaline material-containing reagent a first reagent containing the nonionic surfactant and the chaotropic agent and a second reagent containing the alkaline material.

The "second treatment step" includes mixing the sample obtained in the first treatment step with an acid material-containing reagent as described herein to neutralize the alkalinity of the sample obtained in the first treatment step. The pH of the sample obtained in the second treatment step is preferably such that it has no effect on the forming step described below. More specifically, the pH of the sample obtained in the second treatment step is preferably from 6.5 to 8. The treatment time and the treatment temperature in the second treatment step is not particularly restricted and may be appropriately determined depending on the conditions for the mixing of the sample and the reagent. Specifically, the second treatment step may be performed at a treatment temperature of 20 to 30°C for a treatment time of 3 to 15 minutes.

Examples of the "acid material" include, but are not limited to, acetic acid, lactic acid, citric acid, malic acid, succinic acid, phosphoric acid, formic acid, fumaric acid, tartaric acid, hydrochloric acid, and sulfuric acid. As described herein, the acid material is preferably citric acid. The concentration of the acid material in the acid material-containing reagent used in the second treatment step is not particularly restricted and may be appropriately controlled depending on the acid material used or the alkaline material used in the first step. For example, when the acid material is citric acid, the concentration of the acid material in the acid material-containing reagent is preferably from 0.1 to 0.3 M.

The sample suspected of containing HCV is diluted in the first and second treatment steps. The dilution of the sample suspected of containing HCV in the first and second treatment steps may be appropriately controlled depending on the type of the sample. For example, when the sample suspected of containing HCV is blood serum, the blood serum is preferably diluted 3- to 5-fold in the first and second treatment steps in order to reduce the effect of blood serum components on the immunoassay and to make the concentration of the HCV core protein detectable.

The detection of hepatitis C virus by immune complex transfer assay may be performed by any immunoassay method of detecting an HCV core protein with known particles. The particles may also be any known particles used in immunoassay techniques. Examples of the particles include magnetic particles, latex particles, red blood cells, and gelatin particles. Magnetic particles are preferred. The magnetic particles may be any particles that contain a magnetic material as a base material and are used in conventional immunoassays. For example, known magnetic particles are produced with a base material of Fe₂O₃ and/or Fe₃O₄, cobalt, nickel, ferrite, magnetite, or any other magnetic material.

When hepatitis C virus is detected by an immunoassay using magnetic particles, a complex comprising an HCV core protein, an anti-HCV core protein antibody, and magnetic particles may be formed in a liquid phase, and the complex may be separated from the liquid phase by magnetic separation so that the HCV core protein can be detected. More specifically, a complex comprising an HCV core protein, a labeled anti-HCV core protein antibody (hereinafter also simply referred to as "labeled antibody"), and magnetic particles is formed in a liquid phase. The complex in the liquid phase is then separated from the liquid phase by magnetic separation. The separated complex is then dispersed in a liquid phase, and the label of the complex is measured. Based on the result of the measurement, it can be determined whether hepatitis C virus is present or absent in the sample.

In an embodiment of the invention, the sample obtained in the second treatment step may be used to form a complex comprising an HCV core protein, a labeled antibody, and magnetic particles. In this case, the second treatment step may be followed by forming, on particles, a complex comprising a labeled antibody and an HCV core protein contained in the sample obtained in the second treatment step.

The "labeled antibody" may be any antibody labeled with a known label generally used in immunoassay techniques. The labeled antibody is bound to the HCV core protein by an antigen-antibody reaction. For example, the label may be an enzyme, a fluorescent material, a radioisotope, or the like. Examples of the enzyme include alkaline phosphatase, peroxidase, glucose oxidase, tyrosinase, and acid phosphatase. Examples of the fluorescent material include fluorescein isothiocyanate (FITC), green-fluorescent protein (GFP), and luciferin. Examples of the radioisotope include ¹²⁵I, ¹⁴C, ³²P, and so on. The label is preferably an enzyme.

When the label is an enzyme, the substrate for the enzyme may be appropriately selected from known substrates depending on the enzyme to be used as the label. For example, when alkaline phosphatase is used as the enzyme, examples of the substrate include a chemiluminescent substrate such as CDP-Star® (disodium 4-chloro-3-(methoxyspiro{1,2-dioxetane-3,2'-(5'-chloro)tricyclo[3.3.1.1^{3,7}]decane}-4-yl)phenyl phosphate) or CSPD® (disodium 3-(4-methoxyspiro{1,2-dioxetane-3,2-(5'-chloro)tricyclo[3.3.1.1^{3,7}]decane}-4-yl)phenyl phosphate); a luminescent substrate such as p-nitrophenyl phosphate, 5-bromo-4-chloro-3-indolyl phosphate (BCIP), 4-nitro blue tetrazolium chloride (NBT), or iodonitrotetrazolium (INT); a fluorescent substrate such as 4-methylumbelliferyl phosphate (4-MUP); and a chromogenic substrate such as 5-bromo-4-chloro-3-indolyl phosphate (BCIP), disodium 5-bromo-6-chloro-indolyl phosphate, or p-nitrophenyl phosphate.

The labeled antibody may be prepared by any method known in the art. For example, a known method includes attaching the label to the antibody through the thiol group (-SH) of the antibody. More specifically, a functional group capable of reacting with the thiol group, such as a maleimide group, may be introduced into the label, and then the label may be allowed to react with the antibody so that the antibody can be labeled with the label.

The complex comprising the labeled antibody and the HCV core protein contained in the sample obtained in the second treatment step can be formed on particles by bringing, in a liquid phase, the labeled antibody into contact with magnetic particles and the HCV core protein contained in the sample obtained in the second treatment step. As a result, a complex comprising the HCV core protein, the labeled antibody, and the magnetic particles is formed. In this process, the labeled antibody is bound to the HCV core protein by an antigen-antibody reaction to form a labeled antibody-HCV core protein complex. The labeled antibody-HCV core protein complex can be further bound to magnetic particles through a material capable of binding to the HCV core protein complex and the magnetic particles. For example, the material capable of binding to the HCV core protein complex and the magnetic particles may be a particle-binding antibody capable of binding to the HCV core protein and the magnetic particles. When a particle-binding antibody capable of binding to the HCV core protein and the magnetic particles is used, a labeled antibody-HCV core protein-particle-binding antibody complex is formed on the magnetic particles.

The "particle-binding antibody capable of binding to an HCV core protein and particles" may be any antibody that is capable of binding to particles and can be bound to any other epitope of the HCV core protein than that for the labeled antibody by an antigen-antibody reaction. The antibody capable of binding to particles can be prepared by methods known in the art. For example, a particle binding site may be attached to an antibody, and a binding material capable of binding to the particle binding site may be immobilized on particles, so that an antibody capable of binding to the particles can be prepared. In other words, the binding abilities of the particle binding site and the binding material can be used to make the antibody capable of binding to the particles.

The particle binding site and the binding material may be any combination of materials capable of specifically binding to each other in the forming step. Examples of such a combination include a combination of biotin and any of avidins, a combination of a hapten and an anti-hapten antibody, a combination of nickel and a histidine tag, and a combination of glutathione and glutathione-S-transferase. The combination of the particle binding site and the binding material is preferably a combination of biotin and any of avidins. Each material for use in forming the combination of the particle binding site and the binding material may be used for any one of the particle binding site and the binding material. In a more preferred combination, the particle binding site comprises biotin, and the binding material comprises any of avidins. As used herein, the term "avidins" is intended to include avidin and streptavidin.

The particle binding site may be attached to the antibody by any method known in the art. For example, when the particle binding site comprises biotin, biotin may be attached to the antibody through a group reactive with an amino or thiol group present in the antibody. The group reactive with an amino group may be an N-hydroxysuccinimide (NHS) group, and the group reactive with a thiol group may be a maleimide group.

The binding material may also be immobilized on the particles by any method known in the art. For example, the immobilization may be performed by a physical adsorption method, a covalent binding method, an ion binding method, or any combination thereof. For example, when the binding material is any of avidins, avidins may be directly immobilized on the particles by physical adsorption. Alternatively, particles coupling with a material capable of binding to avidins, such as biotin, may be attached to the avidins, so that avidins can be immobilized on the particles. The method described in JP-A No. 2006-226689 may also be used to immobilize avidins on the particles. Commercially available particles to which avidins are attached may also be used. For example, particles to which avidins are attached may also be purchased from JSR Corporation or Dynal Biotech Ltd.

The complex comprising the HCV core protein, the labeled antibody, and the magnetic particles in the liquid phase is then separated from the liquid phase by magnetic separation. More specifically, a magnet is brought close to the wall surface of the container that contains the liquid containing the complex comprising the HCV core protein, the labeled antibody, and the magnetic particles, so that the complex is fixed on the wall surface of the container using the magnet. The liquid phase is then removed by suction while the complex is fixed on the wall surface of the container, so that the separating step is completed. The separation can remove components unnecessary for the measurement (described below) of the label of the complex or components having an adverse effect on the measurement (described below) of the label of the complex, such as the nonionic surfactant and the chaotropic agent present in the liquid phase, and the labeled antibody not used in the formation of the complex.

The complex comprising the HCV core protein, the labeled antibody, and the magnetic particles may also be washed when separated from the liquid phase by magnetic separation. For example, a washing liquid may be added to the separated complex to form a suspension of the complex, and the complex may be separated from the washing liquid by magnetic separation, so that the complex can be cleaned. The washing step can further remove components unnecessary for the measurement of the label of the complex or components having an adverse effect on the measurement of the label of the complex. The washing method is also known in the field of immunoassays using magnetic particles. The washing liquid is preferably a buffer solution having no effect on the complex comprising the HCV core protein, the labeled antibody, and the magnetic particles. In particular, the washing liquid is preferably a surfactant-containing buffer solution. More specifically, examples of the washing liquid include TBS-T (0.05% Tween 20) and PBS-T (0.05% Tween 20). A commercially available washing liquid such as HISCL washing liquid (manufactured by SYSMEX CORPORATION) may also be used.

The separated complex is then dispersed in a liquid phase, and the label of the complex is measured. The separated complex may be dispersed in a liquid phase such as a substrate solution or a buffer solution. The label may be measured by any appropriate measurement method depending on the type of the label used to form the labeled antibody. For example, when the label is an enzyme, light or color generated by reaction of the enzyme with a substrate may be measured using an appropriate device. Such a device may be a spectrophotometer, a luminometer or the like. When the label is a radioisotope, the measurement may be performed using a known conventional device such as a scintillation counter.

Based on the result of the measurement, it is determined whether hepatitis C virus is present or absent in the sample. The determination of whether hepatitis C virus is present or absent in the sample is made based on the result of the measurement. Specifically, if the label derived from the complex comprising the HCV core protein, the labeled antibody and the magnetic particles is detected in the measurement, it is determined that HCV is present in the sample. On the other hand, if the label derived from the complex comprising the HCV core protein, the labeled antibody and the magnetic particles is not detected in the measurement, it is determined that HCV is absent in the sample. For example, the measured value obtained by the measurement may be compared with the predetermined threshold value. If the measured value is equal to or higher than the threshold value, it may be determined that HCV is present in the sample. On the other hand, if the measured value is less than the threshold value, it may be determined that HCV is absent in the sample. The threshold value may be appropriately defined by known methods. For example, a method of defining the threshold value may include: obtaining a measured value for each of a plurality of samples already known to contain HCV and a measured value for each of a plurality of samples already known not to contain HCV; then defining the threshold value as a median that can divide the measured values into two groups: a group of the HCV-containing samples and a group of the HCV-free samples.

While an immunoassay using magnetic particles has been described for the detection of hepatitis C virus, the method of pretreating a sample for the detection of an HCV core protein may also be applied to other immune complex transfer assay techniques using particles as a solid phase. Other particles than magnetic particles include latex particles, red blood cells, and gelatin particles. When particles other than magnetic particles are used, centrifugal separation may be used in place of magnetic separation to separate the complex from the liquid phase.

In an embodiment of the invention, a method for determining whether hepatitis C virus is present or absent in a sample includes: a first treatment step of treating a sample suspected of containing hepatitis C virus with an alkaline material-containing reagent, which contains an alkaline material, a nonionic surfactant and a chaotropic agent; a second treatment step of treating the sample obtained in the first treatment step with an acid material-containing reagent which contains an acid material and a reducing agent, wherein the reducing agent is at least one selected from mercaptoethylamine, mercaptoethanol, dithiothreitol, cysteine, dithioerythritol, sodium borohydride, and phosphine; a step of forming, on particles, a complex comprising an HCV core protein-binding labeled antibody and an HCV core protein contained in the sample obtained in the second treatment step; a step of separating the particles in the sample from other components in the sample after the forming step; transferring the complex formed on the particles obtained in the separating step to a solid phase other than the particles; a step of measuring the label of the complex obtained in the transferring step and transferred to the solid phase other than the particles; and a step of determining whether hepatitis C virus is present or absent in the sample, based on the result of the measuring step, wherein at least one of the reagents used in the first and second treatment steps contains a reducing agent.

The "particles" may be any known particles used in immunoassay techniques. Specifically, the particles may be the same as those described above.

In the forming step according to this embodiment, a labeled antibody, particles, and an HCV core protein contained in the sample obtained in the second treatment step are brought into contact with one another, so that a complex comprising the labeled antibody and the HCV core protein is formed on the particles. In this step, the labeled antibody is bound to the HCV core protein by an antigen-antibody reaction to form a labeled antibody-HCV core protein complex. The labeled antibody-HCV core protein complex can be further bound to particles through a material capable of binding to the HCV core protein complex and the particles. As a result, a complex comprising the labeled antibody and the HCV core protein can be formed on the particles. For example, the material capable of binding to the HCV core protein complex and the particles may be a particle-binding antibody capable of binding to the HCV core protein and the particles. When a particle-binding antibody capable of binding to the HCV core protein and the particles is used, a labeled antibody-HCV core protein-particle-binding antibody complex is formed on the particles.

In an embodiment of the invention, the method of determining whether hepatitis C virus is present or absent in a sample includes separating the particles in the sample from other components in the sample (hereinafter also referred to as the separating step) after the forming step. The particles separated by the separating step contain the complex comprising the labeled antibody and the HCV core protein. The separating step can remove, from the particles in the sample, components unnecessary for the measuring step described below or components having an adverse effect on the measuring step described below, such as the nonionic surfactant, the chaotropic agent, and the labeled antibody not used in the formation of the complex. In the separating step, the method of separating the particles in the sample from other components is known in the field of immunoassays using particles and may be appropriately selected depending on the particles used. For example, when the particles used are magnetic particles, the particles in the sample can be separated from other components in the sample by magnetic separation. More specifically, a magnet is brought close to the wall surface of the container containing the sample obtained in the second treatment step, so that the particles in the sample are fixed on the wall surface of the container using the magnet. The liquid phase is then removed by suction while the particles are fixed on the wall surface of the container, so that the separating step is completed. When the particles used are gelatin particles or latex particles, the particles in the sample can be separated from the other components in the sample by centrifugal separation. More specifically, the sample obtained in the second treatment step is centrifuged so that the particles are precipitated by the centrifugal force. The resulting supernatant is removed by suction, so that the separating step is completed.

The separating step may also include a washing step. The washing step may include adding a washing liquid to the particles separated by the separating step to suspend the particles and further separating the particles from the washing liquid. The washing step can further remove components unnecessary for the measuring step or components having an adverse effect on the measuring step. Like the separating step, the washing step is also known in the field of immunoassays using particles and may be appropriately selected depending on the particles used. For example, when the particles used are magnetic particles, the particles suspended in the washing liquid can be separated from the washing liquid using magnetic separation. As described above, when the particles used are gelatin particles or latex particles, the particles suspended in the washing liquid can be separated from the washing liquid using centrifugal separation. The washing liquid is preferably a buffer solution having no effect on the complex formed on the particles. In particular, the washing liquid is preferably a surfactant-containing buffer solution. More specifically, examples of the washing liquid include TBS-T (0.05% Tween 20) and PBS-T (0.05% Tween 20). A commercially available washing liquid such as HISCL washing liquid (manufactured by SYSMEX CORPORATION) may also be used.

In an embodiment of the invention, the complex formed on the particles obtained in the separating step is transferred to a solid phase other than the particles (hereinafter also referred to as the transferring step). The complex formed on the particles may be transferred to a solid phase other than the particles, based on a known method. In the forming step, for example, the particles are bound to the complex comprising the labeled antibody and the HCV core protein through binding that can be dissociated using a dissociating agent. Subsequently, the dissociating agent is used to dissociate the complex comprising the labeled antibody and the HCV core protein from the particles, which have been separated by the separating step. The dissociated complex is then brought into contact with a solid phase capable of binding to the complex, so that the complex comprising the labeled antibody and the HCV core protein is transferred to the solid phase other than the particles.

The "solid phase other than the particles" may be any solid phase that is different from the particles used in the forming step. Examples of the solid phase material include polymer materials such as latex, rubber, polyethylene, polypropylene, polystyrene, styrene-butadiene copolymers, polyvinyl chloride, polyvinyl acetate, polyacrylamide, polymethacrylate, styrene-methacrylate copolymers, poly(glycidyl methacrylate), acrolein-ethylene glycol dimethacrylate copolymers, polyvinylidene difluoride (PVDF), and silicone; agarose; gelatin; red blood cells; and inorganic materials such as silica gel, glass, inert alumina, and magnetic materials. These materials may be used singly or in combination of two or more thereof. The solid phase may also have any shape generally used in immunoassays. For example, the solid phase may be in the form of a microtiter plate, a test tube, beads, particles, or nanoparticles.

The "dissociating agent" may be of any type, which may be appropriately selected depending on the type of the binding between the particles and the complex comprising the labeled antibody and the HCV core protein. For example, when the particles and the complex bind through physical adsorption, a surfactant may be used as the dissociating agent. Alternatively, when the particles and the complex bind through hapten-anti-hapten antibody binding, a hapten or a hapten derivative may be used as the dissociating agent. When the particles and the complex bind through ion binding, an ion-containing solution may be used as the dissociating agent. When the particles and the complex bind through dissociable ligand-receptor binding, a ligand or a ligand analogue may be used as the dissociating agent. When the particles and the complex bind through dissociable lectin-sugar chain binding, a sugar may be used as the dissociating agent. When the particles and the complex bind through biotin-avidin binding, biotin may be used as the dissociating agent. When the particles and the complex bind through DNA hybridization, the temperature may be raised so that the complex can be released from the particles.

More specifically, in an embodiment of the invention, the particles and the complex may bind through desthiobiotin-anti-biotin antibody binding or DNP-anti-DNP antibody binding. When the particles and the complex bind through desthiobiotin-anti-biotin antibody binding, biotin may be used as the dissociating agent. When the particles and the complex bind through DNP-anti-DNP antibody binding, DNP-Lys may be used as the dissociating agent. In an embodiment of the invention, the particles and the complex preferably bind through DNP-anti-DNP antibody binding.

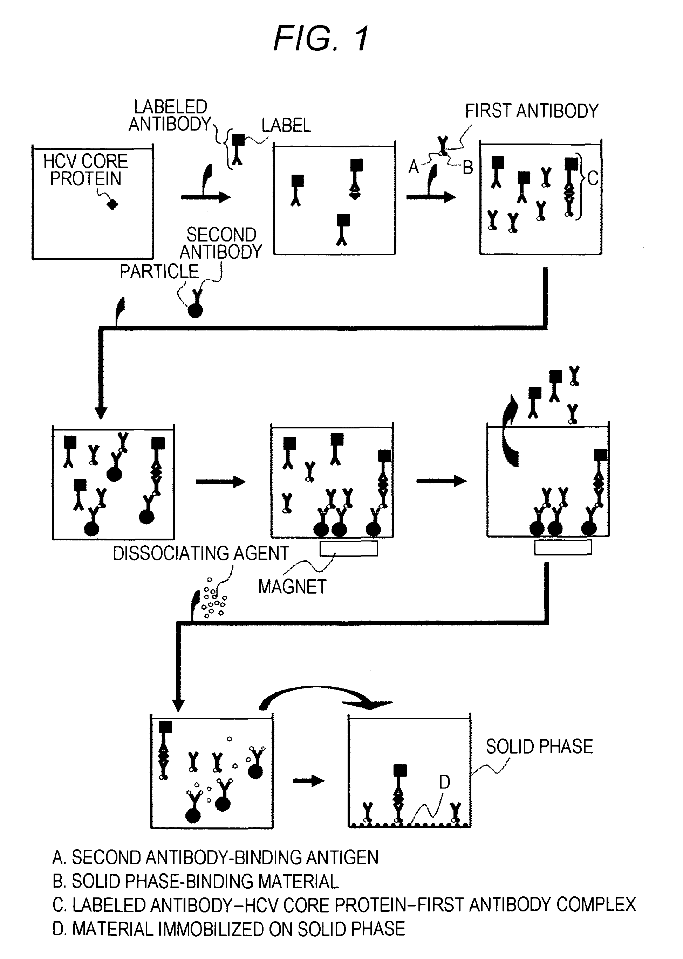

Fig. 1 schematically illustrates a more specific mode of the process according to an embodiment of the invention for measuring the label of the complex transferred to the solid phase other than the particles. First in the forming step, a labeled antibody, an HCV core protein, a first antibody capable of binding to the HCV core protein, and a second antibody that is capable of binding to the first antibody and immobilized on particles are brought into contact with one another, so that a complex comprising the labeled antibody, the HCV core protein, the first antibody, and the second antibody (labeled antibody-HCV core protein-first antibody-second antibody complex) is formed on the particles. Subsequently, the binding between the first and second antibodies in the complex formed on the particles is dissociated, and subsequently, the dissociated complex comprising the labeled antibody, the HCV core protein, and the first antibody (labeled antibody-HCV core protein-first antibody complex) is transferred from the particles to a solid phase on which a material capable of binding to the first antibody is immobilized. The label of the labeled antibody in the complex transferred to the solid phase is then measured. More specifically, the first antibody is modified with an antigen capable of being recognized by the second antibody (a second antibody-binding antigen) and with a material capable of binding to the material immobilized on the solid phase (a solid phase-binding material). The binding between the first and second antibodies can be dissociated by allowing the antigen capable of being recognized by the second antibody to act as a dissociating agent on the particles on which the labeled antibody-HCV core protein-first antibody-second antibody complex is formed. The binding between the antigen serving as the dissociating agent and the second antibody competes with the binding between the first and second antibodies complex, so that the labeled antibody-HCV core protein-first antibody complex can be dissociated from the particles. In addition, the first antibody is modified with a material capable of binding to the material immobilized on the solid phase (solid phase-binding material). Thus, the labeled antibody-HCV core protein-first antibody complex can be transferred to the solid phase by bringing it into contact with the solid phase. The first antibody can be attached to the solid phase using the same binding abilities as those of the particle binding site and the binding material for the particle-binding antibody described above, which binds to the HCV core protein and the particles. For example, a combination of biotin and any of avidins, a combination of a hapten and an anti-hapten antibody, a combination of nickel and a histidine tag, or a combination of glutathione and glutathione-S-transferase may be used to provide the binding abilities.

The binding between the material immobilized on the solid phase and the material capable of binding to the immobilized material may be any other binding than the binding used between the labeled antibody-HCV core protein complex and the particles. More specifically, examples of such binding include hapten-anti-hapten antibody binding, DNA hybrid binding, ion binding, and biotin-avidin binding. In an embodiment of the invention, the binding between the material immobilized on the solid phase and the material capable of binding to the immobilized material is preferably biotin-avidin binding.

In an embodiment of the invention, the label of the complex transferred to the solid phase other than the particles, which is obtained in the transferring step, is measured (hereinafter also referred to as the measuring step). The measuring step may be performed by any appropriate measurement method depending on the type of the label used in the labeled antibody. For example, when the label is an enzyme, light or color generated by reaction of the enzyme with a substrate may be measured using an appropriate device. Such a device may be a spectrophotometer, a luminometer or the like. When the label is a radioisotope, the measurement may be performed using a known conventional device such as a scintillation counter.

In an embodiment of the invention, the method of determining whether hepatitis C virus is present or absent in the sample is performed based on the result of the measuring step (hereinafter also referred to as the determination step). Specifically, if the label derived from the complex comprising the labeled antibody and the HCV core protein is detected in the measuring step, the determination step determines that HCV is present in the sample. On the other hand, if the label derived from the complex comprising the labeled antibody and the HCV core protein is not detected in the measuring step, the determination step determines that HCV is absent in the sample. For example, the measured value obtained in the measuring step may be compared with the predetermined threshold value. If the measured value is equal to or higher than the threshold value, it can be determined that HCV is present in the sample. On the other hand, if the measured value is less than the threshold value, it can be determined that HCV is absent in the sample. The threshold value may be appropriately defined by known methods. For example, a method of defining the threshold value may include: obtaining a measured value for each of a plurality of samples already known to contain HCV and a measured value for each of a plurality of samples already known not to contain HCV; then defining the threshold value as a median that can divide the measured values into two groups: a group of the HCV-containing samples and a group of the HCV-free samples.

The immunoassay method according to an embodiment of the invention is a method for immunoassay of hepatitis C virus (HCV), including the steps of: forming, on a first solid phase, a complex comprising an HCV core protein, a labeled antibody binding to the HCV core protein, and a first antibody binding to the HCV core protein; dissociating the complex, which is formed on the first solid phase, from the first solid phase to transfer the complex to a second solid phase different from the first solid phase; and measuring the label of the complex transferred to the second solid phase, wherein the labeled antibody and the first antibody bind to different sites of the HCV core protein, and the sites to which they bind do not contain the 49th amino acid of the HCV core protein set forth in SEQ ID NO:2.

In an embodiment of the invention, the labeled antibody has an HCV core protein-binding site that does not contain the 49th amino acid of the HCV core protein set forth in SEQ ID NO:2. In an embodiment of the invention, the binding site of the labeled antibody is preferably the amino acid sequence of amino acids 21 to 30 or amino acids 41 to 48 of SEQ ID NO:2.

In an embodiment of the invention, the first antibody has an HCV core protein-binding site that does not contain the 49th amino acid of the HCV core protein set forth in SEQ ID NO:2. The binding site of the first antibody is preferably the amino acid sequence of amino acids 21 to 30 or amino acids 41 to 48 of SEQ ID NO:2. For example, when the binding site of the labeled antibody used is the amino acid sequence of amino acids 21 to 30 of SEQ ID NO:2, the binding site of the first antibody used may be the amino acid sequence of amino acids 41 to 48 of SEQ ID NO:2. Alternatively, when the binding site of the labeled antibody used is the amino acid sequence of amino acids 41 to 48 of SEQ ID NO:2, the binding site of the first antibody used may be the amino acid sequence of amino acids 21 to 30 of SEQ ID NO:2. In an embodiment of the invention, the binding site of the labeled antibody used is preferably the amino acid sequence of amino acids 21 to 30 of SEQ ID NO:2, and the binding site of the first antibody is preferably the amino acid sequence of amino acids 41 to 48 of SEQ ID NO:2.

In the immunoassay method according to an embodiment of the invention, a complex comprising an HCV core protein, the labeled antibody binding to the HCV core protein, and the first antibody binding to the HCV core protein is formed on a first solid phase (hereinafter also referred to as the forming step).

The first solid phase may be any general solid phase used in conventional immunoassay techniques. The material for the first solid phase may be the same as the material for the "solid phase other than the particles" described above.

As described herein, the first solid phase is preferably particles. The particles may be any known particles used in immunoassay techniques. The particles are specifically the same as those described above.

The labeled antibody is bound to the HCV core protein by an antigen-antibody reaction to form a labeled antibody-HCV core protein complex. In addition, the first antibody capable of binding to the HCV core protein is bound to the labeled antibody-HCV core protein complex by an antigen-antibody reaction to form a labeled antibody-HCV core protein-first antibody complex. Alternatively, the first antibody may be bound to the HCV core protein by an antigen-antibody reaction to form an HCV core protein-first antibody complex, and the labeled antibody may be bound to the HCV core protein-fist antibody complex to form a labeled antibody-HCV core protein-first antibody complex.

In the forming step as described herein, the labeled antibody-HCV core protein-first antibody complex may be bound to the first solid phase, so that the complex comprising the labeled antibody, the HCV core protein, and the first antibody is formed on the first solid phase.

The binding between the labeled antibody-HCV core protein-first antibody complex and the first solid phase may be any type of binding through which the labeled antibody-HCV core protein-first antibody complex can be attached to the first solid phase and which can be dissociated in the transferring step described below. For example, the labeled antibody-HCV core protein-first antibody complex may be bound to the first solid phase by physical adsorption. Alternatively, the labeled antibody-HCV core protein-first antibody complex may be bound to particles by ion bonding. A first solid phase-binding site may also be attached to the first antibody, and a binding material capable of binding to the first solid phase-binding site may be immobilized on the first solid phase, so that the labeled antibody-HCV core protein-first antibody complex can be bound to the first solid phase. In an embodiment of the invention, the labeled antibody-HCV core protein-first antibody complex is preferably bound to the first solid phase through the binding between the first solid phase-binding site and the binding material.

The first solid phase-binding site and the binding material may be any combination of materials capable of specifically binding to each other and capable of being dissociated under predetermined conditions. Examples of such a combination of materials include a combination of a ligand and a receptor, a combination of lectin and a sugar chain, a combination of biotin and avidin, a DNA hybrid, and a combination of an antigen and an antibody (second antibody). In an embodiment of the invention, the combination of materials is preferably a combination of an antigen and an antibody (second antibody).

The combination of an antigen and an antibody (second antibody) may a combination of a hapten and an anti-hapten antibody, a combination of desthiobiotin and an anti-biotin antibody, a combination of DNP and an anti-DNP antibody, or the like. In an embodiment of the invention, the combination of an antigen and an antibody (second antibody) is preferably a combination of DNP and an anti-DNP antibody.

In the immunoassay method according to an embodiment of the invention, the complex (labeled antibody-HCV core protein-first antibody complex) formed on the first solid phase is dissociated from the first solid phase and transferred to a second solid phase different from the first solid phase (hereinafter also referred to as the transferring step).

The method for dissociating the labeled antibody-HCV core protein-first antibody complex from the first solid phase is not restricted and may be appropriately selected depending on the type of the binding between the labeled antibody-HCV core protein-first antibody complex and the first solid phase. For example, when the binding is physical absorption binding, a surfactant may be used to dissociate the labeled antibody-HCV core protein-first antibody complex. When the binding is ion binding, an ion-containing solution may be used to dissociate the labeled antibody-HCV core protein-first antibody complex. The binding between the first solid phase-binding site and the binding material may be dissociated by any method that is appropriately selected depending on the combination of the first solid phase-binding site and the binding material. For example, when the binding is ligand-receptor binding, the ligand or an analog of the ligand may be used to dissociate the labeled antibody-HCV core protein-first antibody complex. When the binding is lectin-sugar chain binding, a sugar may be used to dissociate the labeled antibody-HCV core protein-first antibody complex. When the binding is biotin-avidin binding, biotin may be used to dissociate the labeled antibody-HCV core protein-first antibody complex. When the binding is DNA hybrid binding, the temperature may be raised to dissociate the labeled antibody-HCV core protein-first antibody complex. When the binding is hapten-anti-hapten antibody binding, the hapten or a derivative of the hapten may be used to dissociate the labeled antibody-HCV core protein-first antibody complex. When the binding is desthiobiotin-anti-biotin antibody binding, biotin may be used to dissociate the labeled antibody-HCV core protein-first antibody complex. When the binding is DNP-anti-DNP antibody binding, DNP-Lys may be used to dissociate the labeled antibody-HCV core protein-first antibody complex.

Subsequently, the dissociated labeled antibody-HCV core protein-first antibody complex is bound to the second solid phase.

The second solid phase may be any general second solid phase used in conventional immunoassay techniques. The material used for the second solid phase may be the same as that for the first solid phase. The shape of the second solid phase may also be the same as that of the first solid phase. In an embodiment of the invention, the second solid phase is preferably a microtiter plate.

The binding between the labeled antibody-HCV core protein-first antibody complex and the second solid phase may be any type of binding different from the binding between labeled antibody-HCV core protein-first antibody complex and the first solid phase. For example, when the binding between the complex and the first solid phase is DNP-anti-DNP antibody binding, the binding between the complex and the second solid phase may be hapten-anti-hapten antibody binding, DNA hybrid binding, ion binding, or biotin-avidin binding. In an embodiment of the invention, when the binding between the complex and the first solid phase is DNP-anti-DNP antibody binding, the binding between the complex and the second solid phase is preferably biotin-avidin binding.

In an embodiment of the invention, the immunoassay method measures the label of the complex transferred to the second solid phase (hereinafter referred to as the measuring step). The measuring step may be performed by any measurement method that is appropriately selected depending on the type of the label used in the labeled antibody. For example, when the label is an enzyme, light or color generated by reaction of the enzyme with a substrate may be measured using an appropriate device. Such a device may be a spectrophotometer, a luminometer, or the like. When the label is a radioisotope, the measurement may be performed using a known conventional device such as a scintillation counter.

In an embodiment of the invention, when the first solid phase is particles, the immunoassay method may further include: a first treatment step of treating a sample suspected of containing HCV with an alkaline material-containing reagent which contains an alkaline material, a nonionic surfactant and a chaotropic agent; a second treatment step of treating the sample (treated with the alkaline material-containing reagent) with an acid material-containing reagent which contains an acid material and a reducing agent, wherein the reducing agent is at least one selected from mercaptoethylamine, mercaptoethanol, dithiothreitol, cysteine, dithioerythritol, sodium borohydride, and phosphine; and a washing step of washing the particles on which the complex is formed, wherein at least the reagents used in the second treatment step contains a reducing agent selected from mercaptoethylamine, mercaptoethanol, dithiothreitol, cysteine, dithioerythritol, sodium borohydride, and phosphine.

The first and second treatment steps are the same as those described above.

The washing step may include collecting the particles dispersed in a liquid, removing the liquid, and then adding a washing liquid so that the particles are dispersed in the liquid again. The washing step can remove components unnecessary for the measuring step or components having an adverse effect on the measuring step, such as the labeled antibody not used in the formation of the labeled antibody-HCV core protein-first antibody complex, the nonionic surfactant, and the chaotropic agent.

The method of collecting the in-solution dispersing particles in the washing step is known in immunoassay techniques with particles and may be appropriately determined depending on the particles used. For example, when the particles used are magnetic particles, the particles dispersed in the liquid can be collected by magnetic separation. More specifically, a magnet is brought close to the wall surface of the container containing the sample obtained in the second treatment step, so that the the particles in the sample are fixed on the wall surface of the container using the magnet. The liquid is then removed by suction while the particles are fixed on the wall surface of the container. When magnetic particles are used as the particles, the particles dispersed in the liquid can be collected as described above. When the particles used are gelatin particles or latex particles, the particles dispersed in the liquid can be collected by centrifugal separation. More specifically, the sample obtained in the second treatment step is centrifuged so that the particles are precipitated by the centrifugal force. The liquid is then removed by suction while the particles are precipitated. When gelatin or latex particles are used as the particles, the particles dispersed in the liquid can be collected as described above.

In the washing step, a washing liquid is added to suspend the particles, so that the particles are dispersed in the liquid again. The washing liquid is preferably a buffer solution having no effect on the complex formed on the particles. In particular, the washing liquid is preferably a surfactant-containing buffer solution. More specifically, examples of the washing liquid include TBS-T (0.05% Tween 20) and PBS-T (0.05% Tween 20). A commercially available washing liquid such as HISCL washing liquid (manufactured by SYSMEX CORPORATION) may also be used.

The washing step may be performed any number of times, as long as there is no effect on the complex formed on the particles. Repeating the washing step can further remove components unnecessary for the measuring step or components having an adverse effect on the measuring step.

As described herein, at least the reagents used in the second treatment step contains a reducing agent selected from mercaptoethylamine, mercaptoethanol, dithiothreitol, cysteine, dithioerythritol, sodium borohydride, and phosphine. The reducing agent contained in at least the second reagent can prevent particle aggregation when the collected particles are dispersed into a liquid again in the washing step.

The reducing agent may be any type of reducing agent selected from mercaptoethylamine, mercaptoethanol, dithiothreitol, cysteine, dithioerythritol, sodium borohydride, and phosphine having no effect on the forming step. As described herein, the reducing agent is preferably capable of dissociating disulfide bonds of proteins. More specifically, the reducing agent may be the same as described above.

The concentration of the reducing agent in the reagent is not restricted and may be appropriately controlled depending on the type of the reducing agent used. For example, when mercaptoethylamine is used as the reducing agent, the concentration of mercaptoethylamine is preferably from 10 to 60 mM.

In an embodiment of the invention, at least one of the reagents used in the first and second treatment steps preferably further contains an inorganic salt. In an embodiment of the invention, such an inorganic salt may be the same as described above.

The concentration of the inorganic salt in the reagent is not restricted and may be appropriately controlled depending on the type of the inorganic salt used. For example, when sodium chloride is used as the inorganic salt, the concentration of sodium chloride is preferably from 0.1 to 1.0 M.

The antibody having an HCV core protein-binding site that does not contain the 49th amino acid of the HCV core protein set forth in SEQ ID NO:2 can be obtained from a hybridoma produced by known methods.
Specifically, a suitable animal (such as mouse or rat) is immunized with a mixture of an HCV core protein and a suitable adjuvant as needed, and antibody producing cells of the animal, such as spleen cells, lymph node cells, or B lymphocytes, are fused with suitable mammal (such as mouse or rat)-derived myeloma cells to form hybridomas. In general, the antibody producing cells and the myeloma cells are derived from the same species of animal.
For example, the cell fusion may be performed by a PEG method in which the antibody cells and the myeloma cells are fused in the presence of polyethylene glycol in a suitable medium. After the cell fusion, hybridomas are selected in a selection medium such as HAT medium and screened for the ability to produce an antibody capable of recognizing the HCV core protein, according to a conventional method (such as enzyme immunoassay (EIA)). Subsequently, a suitable antibody-producing hybridoma is cloned by a conventional method (such as limiting dilution), so that a monoclonal antibody-producing hybridoma is selected.
After a hybridoma is obtained as described above, the antibody produced by the hybridoma is subjected to epitope analysis. In this way, an antibody having an HCV core protein-binding site that does not contain the 49th amino acid of the HCV core protein set forth in SEQ ID NO:2 is successfully obtained.

Methods of preparing hybridomas that produce an HCV core protein-binding antibody are more specifically described below.

### <Expression and purification of immunogen HCV-derived polypeptide>

### (A) Construction of expression plasmid

The plasmid shown below is obtained which contains DNA encoding the amino acid sequence of amino acids 1 to 160 of the HCV core region set forth in SEQ ID NO:1. pUC·pR-160: a plasmid for the HCV core region protein (pR-160) set forth in SEQ ID NO:1.

One µg of DNA for pUC·pR-160 is digested in 20 µl of a restriction enzyme reaction solution (20 mM Tris-HCl (pH 8.5), 10 mM MgCl₂, 1 mM dithiothreitol, 100 mM KCl, 15 units of Bam H1 enzyme, and 15 units of Hind III enzyme) at 37°C for 1 hour and then subjected to 1.0% agarose gel electrophoresis. The gel portion containing DNA fragments of about 480 bp is cut, and a DNA fragment encoding pR-160 is purified from the agarose gel using QIAquick Gel Extraction Kit (manufactured by QIAGEN).

Subsequently, expression vector pQE-30 (manufactured by QIAGEN) is digested in 20 µl of a restriction enzyme reaction solution (20 mM Tris-HCl (pH 8.5), 10 mM MgCl₂, 1 mM dithiothreitol, 100 mM KCl, 15 units of Bam H1 enzyme, and 15 units of Hind III enzyme) at 37°C for 1 hour and then subjected to 1.0% agarose gel electrophoresis. The gel portion containing DNA fragments of about 3,000 bp is cut, and the Bam HI-Hind III-treated vector DNA is purified from the agarose gel using QIAquick Gel Extraction Kit (manufactured by QIAGEN).

One µg of the resulting Bam HI-Hind III-treated vector DNA and about 0.3 pmol of the pR-160-encoding DNA fragment are subjected to ligation reaction using DNA Ligation Kit Ver. 2.1 (manufactured by TAKARA BIO INC.).

Escherichia coli JM-109 (manufactured by TAKARA BIO INC.) is transformed using 10 µl of the reaction solution obtained by the ligation reaction of the pR-160-enconding DNA fragment. The sensitive E. coli line used in the transformation is E. coli JM109 Competent Cells (manufactured by TAKARA BIO INC.). The transformed E. coli is applied to 100 µg/ml ampicillin-containing LB plates (1% tryptone, 1.0% NaCl, 0.5% yeast extract, 1.5% agar) and incubated overnight at 37°C. A platinum loop of bacterial cells collected from colonies grown on the plate are transferred to a 100 µg/ml ampicillin-containing LB medium (1% tryptone, 1.0% NaCl, 0.5% yeast extract) and cultured overnight at 37°C. The bacterial cells are collected by centrifuging 1.5 ml of the bacterial culture medium, and plasmid DNA minipreparation is performed using QIAprep Spin Miniprep Kit (manufactured by QIAGEN).

One µg of the resulting ligated DNA is digested in 20 µl of a restriction enzyme reaction solution (20 mM Tris-HCl (pH 8.5), 10 mM MgCl₂, 1 mM dithiothreitol, 100 mM KC1, 15 units of Bam H1 enzyme, and 15 units of Hind III enzyme) at 37°C for 1 hour and then subjected to 1.0% agarose gel electrophoresis, so that a pQE-30pR-160 expression plasmid that forms a Bam HI-Hind III fragment of about 480 bp is selected.

### (B) Expression and purification of HCV core-derived polypeptide (pR-160) encoded by pQE-30pR-160

The E. coli JM109 cells containing the pQE-30pR-160 expression plasmid are inoculated into 10 ml of a 100 µg/ml ampicillin-containing LB medium and cultured overnight at 37°C. The whole of the culture medium is transferred to 200 ml of a 100 µg/ml ampicillin-containing LB medium and cultured at 37°C. Isopropyl-β-D-thiogalactoside is added in such an amount that it can have a final concentration of 1 mmol/ml at an OD600 of 0.5 to 0.7, and the culture is further continued overnight at 37°C. The bacterial cells are collected by centrifuging the culture medium.

Subsequently, the bacterial pellet is treated with a surfactant.
Two ml of BugBuster Reagent (manufactured by Novagen) (room temperature) is added to about 1 g (wet weight) of the bacterial cells, and the cells are suspended without foaming. The suspension is incubated for 10 to 20 minutes with gentle shaking at low speed using a rotary shaker. The suspension is centrifuged at 7,500 rpm for 10 minutes, and the supernatant is removed. After the centrifugation, 1.8 ml of BugBuster Reagent (room temperature) is added to the insoluble cell residue, and resuspension is performed without foaming. Lysozyme is added to the suspension so as to have a final concentration of 200 µg/ml. The suspension is incubated for 5 minutes with gentle shaking at low speed using a rotary shaker. The suspension is then centrifuged at 7,500 rpm for 10 minutes, and the supernatant is removed.

Solubilization and purification of the insoluble fraction are then performed. After the centrifugation, 1 ml of 8 M urea, 0.1 M NaH₂PO₄, 0.01 M Tris-Cl (pH=8.0) is added to the resulting insoluble fraction, and resuspension is performed. The suspension is centrifuged at 7,500 rpm for 10 minutes, and the supernatant is removed.
After the centrifugation, 1 ml of 8 M urea, 0.1 M NaH₂PO₄, 0.01 M Tris-Cl (pH=9.0) is added to the insoluble fraction, and resuspension is performed. The suspension is centrifuged at 7,500 rpm for 10 minutes, and the supernatant is removed.
After the centrifugation, 1 ml of 8 M urea, 0.1 M NaH₂PO₄, 0.01 M Tris-Cl (pH=10.0) is added to the insoluble fraction, and resuspension is performed. The suspension is centrifuged at 7,500 rpm for 10 minutes, and the supernatant is removed.
After the centrifugation, 1 ml of 8 M urea, 0.1 M NaH₂PO₄, 0.01 M Tris-Cl (pH=11.0) is added to the insoluble fraction, and resuspension is performed. The suspension is centrifuged at 7,500 rpm for 10 minutes, and the supernatant is removed.
After the centrifugation, 1 ml of 8 M urea, 0.1 M NaH₂PO₄, 0.01 M Tris-Cl (pH=12.0) is added to the insoluble fraction, and the HCV-derived polypeptide (pR-160) is solubilized and extracted.
The polypeptide is purified by metal chelate affinity chromatography using nickel-charged agarose gel, and pR-160 is obtained from the solubilized extract.

### <Preparation of hybridoma>

### (A) Immunization of mice

One hundred µl of Freund's complete adjuvant (FCA) is mixed with 100 µl of a phosphate buffer solution (PBS) containing 100 µg to 1,000 µg of the pR-160 antigen, and the mixture is emulsified to form 200 µl of an FCA pR-160 antigen solution. Two hundred µl of an FIA pR-160 antigen solution is also prepared using the same process, except that Freund's incomplete adjuvant (FIA) is used in place of FCA.

Primary immunization is performed by intraperitoneally administering 200 µl of the FCA pR-160 antigen solution into 7-8 week old female Balb/c mice. After the primary immunization, booster immunization is performed every 2-3 weeks using 200 µl of the FIA pR-160 antigen solution. Ten days and three days before spleen cell harvest, 200 µl of the pR-160 antigen is intravenously administered. Three days after the final administration, spleen cells are isolated and then fused with P3X63-Ag8·653 mouse myeloma cells by PEG method to produce hybridoma cells.

### (B) Hybridoma culture

Hybridoma cells are suspended at a concentration of 2.5x10⁶ cells/ml in a HAT medium, and the suspension is dispensed into each well of a 96-well plate (manufactured by Corning Incorporated, hereinafter referred to as "culture plate") at a density of 2.5x10⁵ cells/well. The culture plate is allowed to stand in an 8% CO₂ incubator at 37°C so that hybridoma culture is started. After culture for at least 10 days to produce hybridoma colonies, screening is performed for monoclonal antibody-producing hybridoma cells.

### (C) Hybridoma screening

The pR-160 antigen is added at a concentration of 0.5 µg/ml to a 0.1 M phosphate buffer solution (PBS, pH 7.5) containing 0.1 w/v% NaN₃ to form a pR-160 antigen fixation solution. One hundred µl of the pR-160 antigen fixation solution is dispensed to each well of Immuno Module (manufactured by NUNC A/S.) (hereinafter referred to as the antigen fixation plate). After allowed to stand overnight at 4°C, the plate is washed three times with a PBS buffer solution containing Tween 20 at a concentration of 0.05% (hereinafter referred to as the buffer A). After the washing, 300 µl of PBS containing BSA at a concentration of 1 w/v% (hereinafter referred to as the buffer B) is added to each well of the antigen fixation plate and allowed to stand at 2 to 8°C for at least 4 hours. The antigen fixation plate is stored at 2 to 8°C until use.

The buffer B is removed from the antigen fixation plate. After the removal, 75 µl of the buffer B is added to each well of the antigen fixation plate. The culture supernatant of the hybridoma culture is taken out of each well of the culture plate, and 25 µl of the supernatant is added to each well of the antigen fixation plate. After the addition of the buffer B and the culture supernatant, stirring is performed at room temperature for 1 hour. After the stirring, each well of the antigen fixation plate is washed three times with the buffer A. After the washing, 100 µl of a 10,000-fold, buffer B-diluted solution of a horseradish peroxidase (POD)-labeled, anti-mouse Ig polyclonal antibody (Code No. P0447 manufactured by Dako) is added to each well of the antigen fixation plate and allowed to react at room temperature for 30 minutes. After the reaction, each well of the antigen fixation plate is washed three times with the buffer A. After the washing, 100 µl of a substrate solution containing o-phenylenediamine (OPD) (substrate for POD) is added to each well and allowed to stand at room temperature for 10 minutes. Subsequently, 100 µl of a reaction quenching solution containing 2 N H₂SO₄ is added to each well of the antigen fixation plate, and then the 492 nm absorbance of the reaction solution in each well is measured using a microplate reader (manufactured by Molecular Devices, Inc.).

The hybridomas that were successfully obtained as described above and each produced an antibody capable of binding to the HCV core protein set forth in SEQ ID NO:1 were named HCF4-801 and HE25, respectively. HCF4-801 was received under reference number NITE BP-844 on November 25, 2009 and deposited under accession number NITE BP-844 at Incorporated Administrative Agency, National Institute of Technology and Evaluation, Patent Microorganisms Depositary (NPMD). Hereinafter, the antibody produced by this hybridoma is called HCF4-801 antibody.
HE25 was received under reference number NITE BP-843 on November 25, 2009 and deposited under accession number NITE BP-843 at Incorporated Administrative Agency, National Institute of Technology and Evaluation, Patent Microorganisms Depositary (NPMD). Hereinafter, the antibody produced by this hybridoma is called HE25 antibody.

### Example 1

### <Expression and purification of HCV-derived polypeptide>

### (A) Construction of expression plasmid

Each plasmid shown below was obtained which contained DNA encoding the amino acid sequence of amino acids 1 to 160 of the HCV core region set forth in SEQ ID NO:2, 3, or 4.
pUC·HCV-J1b: a plasmid for the HCV genotype 1b core region protein (HCV-J1b) set forth in SEQ ID NO:2.
pUC·HCV-J1bT49P: a plasmid for the HCV genotype 1b core region protein (HCV-J1bT49P) set forth in SEQ ID NO:3 with the 49th amino acid mutated from threonine to proline.
pUC·HCV-3bNE137: a plasmid for the HCV genotype 3b NE137 core region protein (HCV-3bNE137) set forth in SEQ ID NO:4.

One µg of DNA for pUC·HCV-J1b was digested in 20 µl of a restriction enzyme reaction solution (20 mM Tris-HCl (pH 8.5), 10 mM MgCl₂, 1 mM dithiothreitol, 100 mM KCl, 15 units of Bam H1 enzyme, and 15 units of Hind III enzyme) at 37°C for 1 hour and then subjected to 1.0% agarose gel electrophoresis. The gel portion containing DNA fragments of about 480 bp was cut, and a DNA fragment encoding HCV-J1b was purified from the agarose gel using QIAquick Gel Extraction Kit (manufactured by QIAGEN).

Subsequently, expression vector pQE-30 (manufactured by QIAGEN) was digested in 20 µl of a restriction enzyme reaction solution (20 mM Tris-HCl (pH 8.5), 10 mM MgCl₂, 1 mM dithiothreitol, 100 mM KCl, 15 units of Bam H1 enzyme, and 15 units of Hind III enzyme) at 37°C for 1 hour and then subjected to 1.0% agarose gel electrophoresis. The gel portion containing DNA fragments of about 3,000 bp was cut, and the Bam HI-Hind III-treated vector DNA was purified from the agarose gel using QIAquick Gel Extraction Kit (manufactured by QIAGEN).

One µg of the resulting Bam HI-Hind III-treated vector DNA and about 0.3 pmol of the HCV-J1b-encoding DNA fragment were subjected to ligation reaction using DNA Ligation Kit Ver. 2.1 (manufactured by TAKARA BIO INC.).

Escherichia coli JM-109 (manufactured by TAKARA BIO INC.) was transformed using 10 µl of the reaction solution obtained by the ligation reaction of the HCV-J1b-enconding DNA fragment. The sensitive E. coli line used in the transformation was E. coli JM109 Competent Cells (manufactured by TAKARA BIO INC.). The transformed E. coli was applied to 100 µg/ml ampicillin-containing LB plates (1% tryptone, 1.0% NaCl, 0.5% yeast extract, 1.5% agar) and incubated overnight at 37°C. A platinum loop of bacterial cells collected from colonies grown on the plate were transferred to a 100 µg/ml ampicillin-containing LB medium (1% tryptone, 1.0% NaCl, 0.5% yeast extract) and cultured overnight at 37°C. The bacterial cells were collected by centrifuging 1.5 ml of the bacterial culture medium, and plasmid DNA minipreparation was performed using QIAprep Spin Miniprep Kit (manufactured by QIAGEN).

One µg of the resulting ligated DNA was digested in 20 µl of a restriction enzyme reaction solution (20 mM Tris-HCl (pH 8.5), 10 mM MgCl₂, 1 mM dithiothreitol, 100 mM KCl, 15 units of Bam H1 enzyme, and 15 units of Hind III enzyme) at 37°C for 1 hour and then subjected to 1.0% agarose gel electrophoresis, so that a pQE-30HCV-J1b expression plasmid that formed a Bam HI-Hind III fragment of about 480 bp was selected.

A pQE-30HCV-J1bT49P expression plasmid and a pQE-30HCV-3bNE137 expression plasmid were selected using pUC·HCV-J1bT49P and pUC·HCV-3bNE137, respectively, by the same procedure as in the case that the pQE-30HCV-J1b expression plasmid was selected using pUC·HCV-J1b.

### (B) Expression and purification of HCV core-derived polypeptide (HCV-J1b) encoded by pQE-30HCV-J1b

The E. coli JM109 cells containing the pQE-30HCV-J1b expression plasmid were inoculated into 10 ml of a 100 µg/ml ampicillin-containing LB medium and cultured overnight at 37°C. The whole of the culture medium was transferred to 200 ml of a 100 µg/ml ampicillin-containing LB medium and cultured at 37°C. Isopropyl-β-D-thiogalactoside was added in such an amount that it could have a final concentration of 1 mmol/ml at an OD600 of 0.5 to 0.7, and the culture was further continued overnight at 37°C. The bacterial cells were collected by centrifuging the culture medium.

Subsequently, the bacterial pellet was treated with a surfactant.
Two ml of BugBuster Reagent (manufactured by Novagen) (room temperature) was added to about 1 g (wet weight) of the bacterial cells, and the cells were suspended without foaming. The suspension was incubated for 10 to 20 minutes with gentle shaking at low speed using a rotary shaker. The suspension was centrifuged at 7,500 rpm for 10 minutes, and the supernatant was removed. After the centrifugation, 1.8 ml of BugBuster Reagent (room temperature) was added to the insoluble cell residue, and resuspension was performed without foaming. Lysozyme was added to the suspension so as to have a final concentration of 200 µg/ml. The suspension was incubated for 5 minutes with gentle shaking at low speed using a rotary shaker. The suspension was then centrifuged at 7,500 rpm for 10 minutes, and the supernatant was removed.

Solubilization and purification of the insoluble fraction were then performed. After the centrifugation, 1 ml of 8 M urea, 0.1 M NaH₂PO₄, 0.01 M Tris-Cl (pH=8.0) was added to the resulting insoluble fraction, and resuspension was performed. The suspension was centrifuged at 7,500 rpm for 10 minutes, and the supernatant was removed.
After the centrifugation, 1 ml of 8 M urea, 0.1 M NaH₂PO₄, 0.01 M Tris-Cl (pH=9.0) was added to the insoluble fraction, and resuspension was performed. The suspension was centrifuged at 7,500 rpm for 10 minutes, and the supernatant was removed.
After the centrifugation, 1 ml of 8 M urea, 0.1 M NaH₂PO₄, 0.01 M Tris-Cl (pH=10.0) was added to the insoluble fraction, and resuspension was performed. The suspension was centrifuged at 7,500 rpm for 10 minutes, and the supernatant was removed.
After the centrifugation, 1 ml of 8 M urea, 0.1 M NaH₂PO₄, 0.01 M Tris-Cl (pH=11.0) was added to the insoluble fraction, and resuspension was performed. The suspension was centrifuged at 7,500 rpm for 10 minutes, and the supernatant was removed.
After the centrifugation, 1 ml of 8 M urea, 0.1 M NaH₂PO₄, 0.01 M Tris-Cl (pH=12.0) was added to the insoluble fraction, and the HCV-derived polypeptide (HCV-J1b) was solubilized and extracted.
The polypeptide was purified by metal chelate affinity chromatography using nickel-charged agarose gel, and HCV-J1b was obtained from the solubilized extract.

HCV-J1bT49P and HCV-3bNE137 were obtained using the pQE-30HCV-J1bT49P expression plasmid and the pQE-30HCV-3bNE137 expression plasmid, respectively, by the same procedure as in the case that HCV-J1b was obtained using pQE-30HCV-J1b.

### Example 2

### Epitope analysis

HCV-J1b and HCV-J1bT49P obtained in Example 1 were used together with the synthetic peptides CDP-2, CDP-2-1, CDP-3, and CDP-3-1 shown in Table 1 in the analysis of the epitopes of the HCF4-801 antibody and the HE25 antibody, which are anti-HCV core protein monoclonal antibodies. CDP-2, CDP-2-1, CDP-3, and CDP-3-1 were each a keyhole limpet hemocyanin-conjugated synthetic peptide, the preparation of which was outsourced to Operon Biotechnologies.

**Table 1: List of the amino acid sequences of HCV core-derived peptides**

| | **Peptide name** | **Amino acid sequence** | **AA location** |
|---|---|---|---|
| **SEQ ID NO:5** | CDP-2 | DVKFPGGGQIVGGVYPRR | 21-40 |
| **SEQ ID NO:6** | CDP-2-1 | DVKFPGGGQ1 | 21-30 |
| **SEQ ID NO:7** | CDP-3 | GPRLGVRATRKYSKRSQPRG | 41-60 |
| **SEQ ID NO:8** | CDP-3-1 | GPRLGVRATR | 41-50 |

### (A) Determination of reactivity of HCF4-801 antibody to CDP-2

CDP-2 was added at a concentration of 0.5 µg/ml to a 0.1 M phosphate buffer solution (PBS, pH 7.5) containing 0.1 w/v% NaN₃ to form a CDP-2 fixation solution. One hundred µl of the CDP-2 fixation solution was dispensed to each well of Immuno Module (manufactured by NUNC A/S.) (hereinafter referred to as the antigen fixation plate). After allowed to stand overnight at 4°C, the plate was washed three times with a PBS buffer solution containing Tween 20 at a concentration of 0.05% (hereinafter referred to as the buffer A). After the washing, 300 µl of PBS containing BSA at a concentration of 1 w/v% (hereinafter referred to as the buffer B) was added to each well of the peptide fixation plate and allowed to stand at 2 to 8°C for at least 4 hours. The antigen fixation plate was stored at 2 to 8°C until use.

The buffer B was removed from the CDP-2 fixation plate. After the removal, 75 µl of the buffer B was added to each well of the antigen fixation plate. The culture supernatant of the HCF4-801 hybridoma culture was taken out of each well of the culture plate, and 25 µl of the supernatant was added to each well of the antigen fixation plate. After the addition of the buffer B and the culture supernatant, stirring was performed at room temperature for 1 hour. After the stirring, each well of the antigen fixation plate was washed three times with the buffer A. After the washing, 100 µl of a 10,000-fold, buffer B-diluted solution of a horseradish peroxidase (POD)-labeled, anti-mouse Ig polyclonal antibody (Code No. P0447 manufactured by Dako) was added to each well of the antigen fixation plate and allowed to react at room temperature for 30 minutes. After the reaction, each well of the antigen fixation plate was washed three times with the buffer A. After the washing, 100 µl of a substrate solution containing o-phenylenediamine (OPD) (substrate for POD) was added to each well and allowed to stand at room temperature for 10 minutes. Subsequently, 100 µl of a reaction quenching solution containing 2 N H₂SO₄ was added to each well of the antigen fixation plate, and then the 492 nm absorbance of the reaction solution in each well was measured using a microplate reader (manufactured by Molecular Devices, Inc.). When the absorbance was 0.15 or more, reactivity was determined to be present, and when the absorbance was less than 0.15, reactivity was determined to be absent.

### (B) Determination of reactivity of HE25 antibody to CDP-2

Reactivity of HE25 antibody to CDP-2 was determined in the same manner as in the case that the reactivity of the HCF4-801 antibody to CDP-2 was determined.

Reactivity of the HCF4-801 antibody and the HE25 antibody to CDP-2-1, CDP-3, CDP-3-1, HCV-J1b, and HCV-J1bT49P was also determined in the same manner as in Example 2(A) and Example 2(B). The results are shown in Table 2.

**Table 2: Presence or absence of reactivity of HCF4-801 and HE25 antibodies to each synthetic polypeptide**

| | HCF4-801 | HE-25 |
|---|---|---|
| CDP2 (A A 21-40) | ○ | × |
| CDP2-1 (A A 21-30) | ○ | × |
| CDP3 (A A 41-60) | × | ○ |
| CDP3-1 (A A 41-50) | × | ○ |
| HCV - J1b (A A 1-160) | ○ | ○ |
| HCV - J1bT49 (A. A. 1-160) | ○ | ○ |

| | | |
|---|---|---|
| ○: Reactivity is present; X: Reactivity is absent. | | |

From Table 2, it is apparent that the HCF4-801 antibody is a monoclonal antibody that recognizes the amino acid sequence of amino acids 21 to 30 of the HCV core region set forth in SEQ ID NO:2. It is also apparent that the epitope for the HE25 antibody, which is reactive to CDP-2 and CDP-3-1, resides in the amino acid sequence of amino acids 41 to 50 of the HCV core region set forth in SEQ ID NO:2. It is also apparent that the epitope does not contain the 49th amino acid of the HCV core region set forth in SEQ ID NO:2, because the HE25 antibody is reactive to HCV-J1bT49P. From these results, it is apparent that the HE25 antibody is a monoclonal antibody that recognizes the amino acid sequence of amino acids 41 to 48 of the HCV core region set forth in SEQ ID NO:2.

### Example 3

### <Genotype reactivity of anti-HCV core monoclonal antibody>

The experiment described below was performed using the HCV core region-derived polypeptides obtained in Example 1. The reagents used in this example are shown below.

### Labeled antibody reagent

An ALP-labeled anti-HCV antibody (ALP-labeled HCF4-801) was used as a labeled antibody. More specifically, the labeled antibody reagent was prepared by a process including converting the antibody into the Fab' by pepsin digestion and reduction and mixing the Fab' and ALP maleimidated with EMCS (N-(6-maleimidocaproyloxy)succinimido) (DOJINDO LABORATORIES) as a crosslinking agent to form the labeled antibody by their reaction. The ALP-labeled antibody prepared by this method was diluted 80-fold with a diluent (25 mM Tris-HCl (pH 7.5), 0.15 M NaCl, 1.0% BSA, 0.02% NaN₃, 1 mM MgCl₂, 0.1 mM ZnCl₂) to form a labeled antibody reagent.

### First antibody reagent

The first antibody used was an antibody modified with biotin and DNP (biotin/DNP-labeled HE25). More specifically, the first antibody reagent was prepared by a process including: adding a biotinylating reagent (EZ-Link Sulfo-NHS-LC-Biotin Reagents, PIERCE) to BSA; then adding a DNP labeling reagent (DNP-X acid SE, ABD Bioquest, Inc.) thereto to form biotin/DNP-labeled BSA; converting the antibody into the Fab' by pepsin digestion and reduction; and then mixing the Fab' and the biotin/DNP-labeled BSA maleimidated with EMCS (N-(6-maleimidocaproyloxy)succinimido) (DOJINDO LABORATORIES) as a crosslinking agent to form the first antibody by their reaction. The first antibody prepared by this method was diluted 50-fold to form the first antibody reagent.

### Magnetic particle reagent

An anti-DNP antibody (DNP-1753) was immobilized as the second antibody on magnetic particles (micromer-M PEG-NH₂, Micromod Partikeltechnologie GmbH) to be used. DNP-1753 used was a monoclonal antibody produced by a hybridoma deposited under accession number NITE BP-845. The hybridoma cells were received under reference number NITE BP-845 on November 25, 2009 at Incorporated Administrative Agency, National Institute of Technology and Evaluation, Patent Microorganisms Depositary (NPMD). Five µl of the magnetic particles were diluted 200-fold with 995 µl of a diluent (25 mM Tris-HCl (pH 7.5), 0.15 M NaCl, 1.0% BSA, 0.02% NaN₃) to form a magnetic particle reagent.

### Solid phase

The solid phase used was a streptavidin-immobilized plate (streptavidin plate). The streptavidin plate was prepared by allowing 100 µl of a solution of 10 µg/ml streptavidin (Wako Pure Chemical Industries, Ltd.) to act on C8 WHITE MAXISORP Plate (manufactured by NUNC A/S.).

### Dissociating agent

DNP-Lys was used as a material for dissociating the binding between the first and second antibodies. DNP-Lys (manufactured by Tokyo Chemical Industry Co., Ltd.) was diluted to 3.0 mM with a diluent (25 mM Tris-HCl (pH 7.5), 0.15 M NaCl, 1.0% BSA, 0.02% NaN₃, 0.5 wt% sodium caseinate) to form a dissociating agent.

The recombinant HCV core antigen prepared in Example 1 was diluted to a concentration of 100 pg/ml with a diluent (25 mM Tris-HCl (pH 7.5), 0.15 M NaCl, 1.0% BSA, 0.02% NaN₃) to form an antigen sample.

Genotype reactivity of the anti-HCV core monoclonal antibody was examined using the recombinant HCV core antigen-containing sample.

### <Forming step>

Fifty µl of the labeled antibody reagent and 125 µl of the sample containing 100 pg/ml of the recombinant HCV core antigen were mixed in a reaction cuvette (reaction cuvette for HISCL, manufactured by SYSMEX CORPORATION) and incubated at room temperature for 5 minutes. Subsequently, 50 µl of the first antibody reagent was added to the reaction cuvette and incubated at room temperature for 5 minutes. Subsequently, 50 µl of the magnetic particle reagent was added to the reaction cuvette and incubated at room temperature for 10 minutes, so that the forming step was completed.

### <Washing step>

The reaction cuvette containing the sample obtained in the forming step was subjected to magnetic separation using a magnetic separator (MiniTube Mag Separator manufactured by Spherotech, Inc.). The supernatant was removed, and 380 µl of a washing buffer (HISCL washing buffer, manufactured by SYSMEX CORPORATION) was added to the reaction cuvette, and washing accompanied by magnetic separation was performed again. After the washing was repeated twice, washing with 190 µl of the washing buffer was performed once, so that the washing step was completed.

### <Transferring step>

Fifty µl of the dissociating agent was added to the reaction cuvette containing the sample obtained in the washing step and incubated at room temperature for 4 minutes. The sample was then subjected to magnetic separation, and the supernatant was transferred to the solid phase and incubated at room temperature for 20 minutes. Subsequently, the supernatant was removed, 300 µl of the washing buffer was added to the solid phase, and washing accompanied by removal of the supernatant was performed again. The washing was repeated five times, so that the transferring step was completed.

### <Measuring step>

Twenty five µl of HISCL R4 reagent (manufactured by SYSMEX CORPORATION) and 25 µl of HISCL R5 reagent were added to the solid phase containing the sample obtained in the transferring step and incubated at 42°C for 5 minutes. The luminescence intensity (counts) was measured by photometry (3 seconds at gain 4095) using FLUOstar OPTIMA (manufactured by BMG LABTECH GmbH), so that the measuring step was completed.

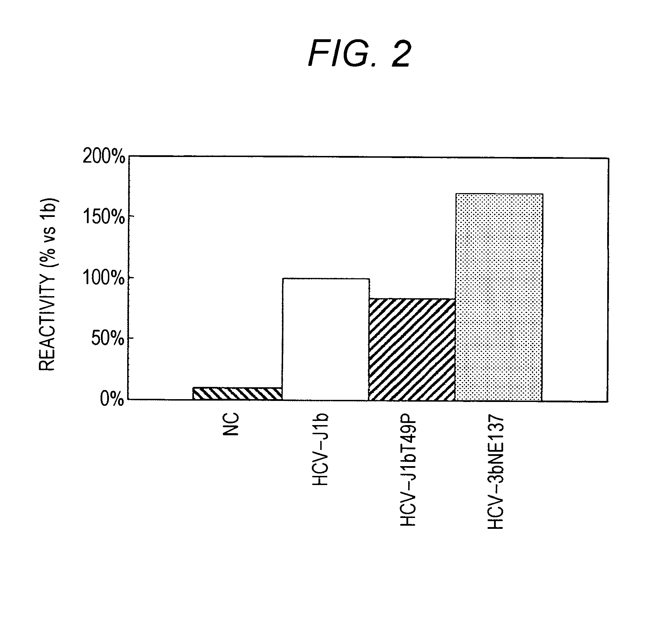

The results are shown in Fig. 2. Fig. 2 shows the reactivities of a negative control (NC), HCV-J1b, HCV-J1bT49P, and HCV-3bNE137. Each reactivity was obtained by calculating the luminescence intensities of the negative control (NC), HCV-J1bT49P, and HCV-3bNE137 with the luminescence intensity of HCV-J1b normalized as 100.

As shown in Fig. 2, HCV-J1bT49P and HCV-3bNE137 were successfully detected at substantially the same level as HCV-J1b and at a higher sensitivity than HCV-J1b, respectively, by the measurement using the anti-HCV core monoclonal antibodies HCF4-801 and HE25. This suggests that the HCV core proteins of HCV-1b variants and HCV-3b can be detected with high sensitivity using the two antibodies having a binding site not containing the 49th amino acid of the HCV core protein set forth in SEQ ID NO:2.

### Example 4

### <Genotype reactivity of anti-HCV core monoclonal antibody>

The experiment described below was performed using HCV-positive serum Nos. 43 to 48 obtained from HCV-positive patients and each having a known HCV genotype and a known HCV-RNA quantity.

The reagents used in this example are shown below.

### First treatment reagent

An aqueous solution containing 6 M of urea as a chaotropic agent, 4% of Brij 35 (manufactured by Sigma-Aldrich Co.) as a surfactant, and 0.45 N of sodium hydroxide as an alkaline material was used as the first treatment reagent.

### Second treatment reagent

The second treatment reagents A, B, and C shown below were prepared, which were each an aqueous solution containing 0.15 M of citric acid as an acid material.

| Second treatment reagent A | | |
|---|---|---|
| | Citric acid | 0.15 M |

| Second treatment reagent B | | |
|---|---|---|
| | Citric acid | 0.15 M |
| | Mercaptoethylamine | 30 mM |

| Second treatment reagent C | | |
|---|---|---|
| | Citric acid | 0.15 M |
| | Mercaptoethylamine | 30 mM |
| | NaCl | 0.6 mM |

The second treatment reagent B contains mercaptoethylamine as a reducing agent. The second treatment reagent C contains mercaptoethylamine as a reducing agent and NaCl as an inorganic salt.

### Labeled antibody reagent

Twenty µl of the same ALP-labeled antibody as used in Example 3 was diluted 80-fold with 980 µl of a diluent (25 mM Tris-HCl (pH 7.5), 0.15 M NaCl, 0.25% BSA, 0.02% NaN₃, 1 mM MgCl₂, 0.1 mM ZnCl₂) to form a labeled antibody reagent.

### First antibody reagent

Twenty five µl of the same first antibody reagent as used in Example 3 was diluted 20-fold with 475 µl of the diluent to form a first antibody reagent.

### Magnetic particle reagent

An anti-DNP antibody (DNP-1753) was immobilized as the second antibody on magnetic particles (Dynabeads M-270, manufactured by Invitrogen Corporation) to be used. DNP-1753 used was a monoclonal antibody produced by a hybridoma deposited under accession number NITE BP-845. The hybridoma cells were received under reference number NITE BP-845 on November 25, 2009 at Incorporated Administrative Agency, National Institute of Technology and Evaluation, Patent Microorganisms Depositary (NPMD). One hundred µl of the magnetic particles were diluted 10-fold with 900 µl of the diluent to form a magnetic particle reagent.

### Solid phase

The same streptavidin plate as in Example 3 was used.

### Dissociating agent

DNP-Lys was used as a material for dissociating the binding between the first and second antibodies. DNP-Lys (manufactured by Tokyo Chemical Industry Co., Ltd.) was diluted to 0.2 mM with a diluent (25 mM Tris-HCl (pH 7.5), 0.6 M NaCl, 0.25% BSA, 0.02% NaN₃) to form a dissociating agent.

### HCV-positive sera

The HCV-positive sera used were World Wide HCV Performance Panel (manufactured by SeraCare) shown in Table 3 below.

**Table 3**

| **Serum No.** | **Sample ID** | **genotype** | **HCV-RNA (IU/mL)** |
|---|---|---|---|
| **43** | WWHV302-01 | 1b | 1.3 × 10⁶ |
| **44** | WWHV302-02 | 1 | 4.3 × 10⁵ |
| **45** | WWHV302-03 | 1b | 1.4 × 10⁵ |
| **46** | WWHV302-04 | 2a | 9.1 × 10⁵ |
| **47** | WWHV302-06 | 3b | 2.4 × 10⁴ |
| **48** | WWHV302-08 | 3a | 7.2 × 10⁵ |

The HCV core protein in each of HCV-positive serum Nos. 43 to 48 shown in Table 3 was detected as described below.

### First treatment step

A mixture of 40 µl of the HCV-positive serum and 60 µl of the first treatment reagent was incubated at room temperature for 8 minutes, so that the first treatment step was completed.

### Second treatment step

Sixty µl of the second treatment reagent was added to the sample obtained in the first treatment step and then incubated at room temperature for 5 minutes, so that the second treatment step was completed. After the second treatment step, it was confirmed that the sample had a pH of 7 to 7.5 using pH test paper (manufactured by Whatman plc.).

### <Forming step>

Seventy five µl of the labeled antibody reagent and 160 µl of the sample obtained in the second treatment step were mixed in a reaction cuvette (reaction cuvette for HISCL, manufactured by SYSMEX CORPORATION) and incubated at room temperature for 10 minutes. Subsequently, 50 µl of the first antibody reagent was added to the reaction cuvette and incubated at room temperature for 10 minutes. Subsequently, 120 µl of the magnetic particle reagent was added to the reaction cuvette and incubated at room temperature for 10 minutes, so that the forming step was completed.

### <Washing step>

The reaction cuvette containing the sample obtained in the forming step was subjected to magnetic separation using a magnetic separator (MiniTube Mag Separator manufactured by Spherotech, Inc.). The supernatant was removed, and 380 µl of a washing buffer (HISCL washing buffer, manufactured by SYSMEX CORPORATION) was added to the reaction cuvette, and washing accompanied by magnetic separation was performed again. After the washing was repeated twice, washing with 190 µl of the washing buffer was performed once, so that the washing step was completed.

### <Transferring step>

Forty µl of the dissociating agent was added to the reaction cuvette containing the sample obtained in the washing step and incubated at room temperature for 4 minutes. The sample was then subjected to magnetic separation, and the supernatant was transferred to the solid phase and incubated at room temperature for 20 minutes. Subsequently, the supernatant was removed, 300 µl of the washing buffer was added to the solid phase, and washing accompanied by removal of the supernatant was performed again. The washing was repeated five times, so that the transferring step was completed.

### <Measuring step>

Twenty µl of HISCL R4 reagent (manufactured by SYSMEX CORPORATION) and 20 µl of HISCL R5 reagent were added to the solid phase containing the sample obtained in the transferring step and incubated at 42°C for 4 minutes. The luminescence intensity (counts) was measured by photometry (3 seconds at gain 4095) using FLUOstar OPTIMA (manufactured by BMG LABTECH GmbH), so that the measuring step was completed.

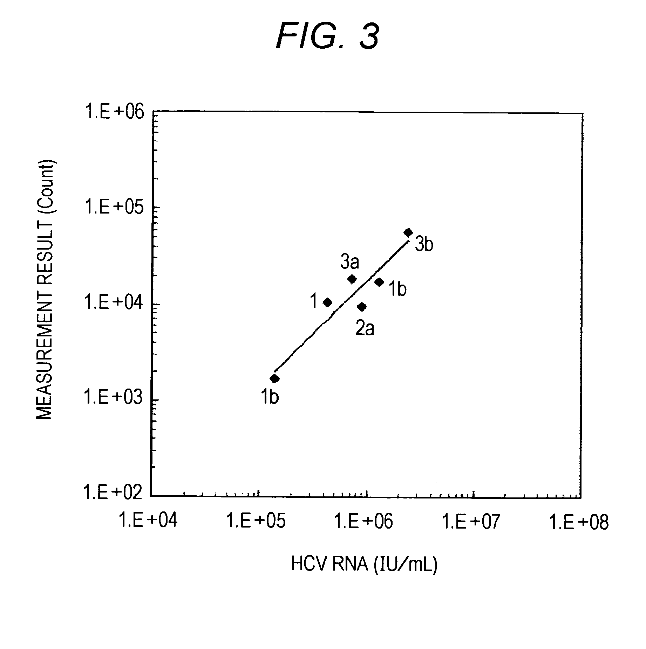

The results are shown in Fig. 3. Fig. 3 shows the relationship between the HCV-RNA quantity and the luminescence intensity.
As shown in Fig. 3, no genotype deviating from the correlation between the HCV-RNA quantity and the luminescence intensity was observed in the measurement using the anti-HCV core monoclonal antibodies HCF4-801 and HE25. This suggests that each HCV genotype core protein can be detected at a similar level using the two antibodies having a binding site not containing the 49th amino acid of the HCV core protein set forth in SEQ ID NO:2.

### Example 5 (045, 064, 069)

### <Determining whether a reducing agent inhibits aggregation of magnetic particles>

The reagents used in this example are shown below.

### First treatment reagent

An aqueous solution containing 6 M of urea as a chaotropic agent, 4% of Brij 35 (manufactured by Sigma-Aldrich Co.) as a surfactant, and 0.45 N of sodium hydroxide as an alkaline material was used as the first treatment reagent.

### Second treatment reagent

The second treatment reagents A, B, and C shown below were prepared, which were each an aqueous solution containing 0.15 M of citric acid as an acid material.

| Second treatment reagent A | | |
|---|---|---|
| | Citric acid | 0.15 M |

| Second treatment reagent B | | |
|---|---|---|
| | Citric acid | 0.15 M |
| | Mercaptoethylamine | 30 mM |

| Second treatment reagent C | | |
|---|---|---|
| | Citric acid | 0.15 M |
| | Mercaptoethylamine | 30 mM |
| | NaCl | 0.6 mM |

The second treatment reagent B contains mercaptoethylamine as a reducing agent. The second treatment reagent C contains mercaptoethylamine as a reducing agent and NaCl as an inorganic salt.

### Labeled antibody reagent

An ALP-labeled anti-HCV antibody (ALP-labeled HCF3-807) was used as a labeled antibody. HCF3-807 used was a monoclonal antibody produced by a hybridoma received under reference number NITE BP-842. The hybridoma cells were received under reference number NITE BP-842 on November 25, 2009 at Incorporated Administrative Agency, National Institute of Technology and Evaluation, Patent Microorganisms Depositary (NPMD). More specifically, the labeled antibody reagent was prepared by a process including converting the antibody into the Fab' by pepsin digestion and reduction and mixing the Fab' and ALP maleimidated with EMCS (N-(6-maleimidocaproyloxy)succinimido) (DOJINDO LABORATORIES) as a crosslinking agent to form the labeled antibody by their reaction. Twenty µl of the ALP-labeled antibody prepared by this method was diluted 50-fold with 980 µl of a diluent (25 mM Tris-HCl (pH 7.5), 0.15 M NaCl, 0.25% BSA, 0.02% NaN₃, 1 mM MgCl₂, 0.1 mM ZnCl₂) to form a labeled antibody reagent.

### First antibody reagent

The first antibody used was an antibody modified with biotin and DNP (biotin/DNP-labeled HCF4-104). HCF4-104 used was a monoclonal antibody produced by a hybridoma received under reference number NITE BP-841. The hybridoma cells were received under reference number NITE BP-841 on November 25, 2009 at Incorporated Administrative Agency, National Institute of Technology and Evaluation, Patent Microorganisms Depositary (NPMD). More specifically, the first antibody reagent was prepared by a process including: adding a biotinylating reagent (EZ-Link Sulfo-NHS-LC-Biotin Reagents, PIERCE) to BSA; then adding a DNP labeling reagent (DNP-X acid SE, ABD Bioquest, Inc.) thereto to form biotin/DNP-labeled BSA; converting the antibody into the Fab' by pepsin digestion and reduction; and then mixing the Fab' and the biotin/DNP-labeled BSA maleimidated with EMCS (N-(6-maleimidocaproyloxy)succinimido) (DOJINDO LABORATORIES) as a crosslinking agent to form the first antibody by their reaction. Five µl of the first antibody prepared by this method was diluted 100-fold with 495 µl of a diluent to form a first antibody reagent.

### Magnetic particle reagent

An anti-DNP antibody (DNP-1753) was immobilized as the second antibody on magnetic particles (Dynabeads M-270, manufactured by Invitrogen Corporation) to be used. DNP-1753 used was a monoclonal antibody produced by a hybridoma received under reference number NITE BP-845. The hybridoma cells were received under reference number NITE BP-845 on November 25, 2009 at Incorporated Administrative Agency, National Institute of Technology and Evaluation, Patent Microorganisms Depositary (NPMD). One hundred µl of the magnetic particles were diluted 10-fold with 900 µl of a diluent to form a magnetic particle reagent.

The magnetic particle aggregation-inhibiting effect of the reducing agent was determined as described below.

### <First treatment step>

A mixture of 40 µl of HCV-negative serum and 60 µl of the first treatment reagent was incubated at room temperature for 8 minutes, so that the first treatment step was completed.

### <Second treatment step>

Sixty µl of each of the second treatment reagents A, B, and C was added to the sample obtained in the first treatment step and then incubated at room temperature for 5 minutes, so that the second treatment step was completed. After the second treatment step, it was confirmed that the sample had a pH of 7 to 7.5 using pH test paper (manufactured by Whatman plc.).

### <Forming step>

Seventy five µl of the labeled antibody reagent and 160 µl of the sample obtained in the second treatment step were mixed in a reaction cuvette (reaction cuvette for HISCL, manufactured by SYSMEX CORPORATION) and incubated at room temperature for 10 minutes. Subsequently, 50 µl of the first antibody reagent was added to the reaction cuvette and incubated at room temperature for 10 minutes. Subsequently, 80 µl of the magnetic particle reagent was added to the reaction cuvette and incubated at room temperature for 10 minutes, so that the forming step was completed.

### <Washing step>

The reaction cuvette containing the sample obtained in the forming step was subjected to magnetic separation using a magnetic separator (MiniTube Mag Separator manufactured by Spherotech, Inc.). Three hundred µl of HISCL washing liquid (manufactured by SYSMEX CORPORATION) was added to the magnetically separated magnetic particles, and the particles were dispersed using a vortex mixer, so that the washing step was completed. Subsequently, the reaction cuvette was allowed to stand, and particle aggregation was evaluated.

In the evaluation of particle aggregation, the particles were visually observed, and the degree of the aggregation was evaluated on the following three criteria.
++: Aggregation was observed.
+: Aggregation was slightly observed.
-: No aggregation was observed.
Table 4 shows the results of the evaluation of aggregation in the samples prepared with the second treatment reagents A, B, and C, respectively, in the reaction cuvette.

**Table 4**

| **Second treatment reagent** | **Evaluation of particle aggregation** |
|---|---|
| A | + + |
| B | - |
| C | - |

As shown in Table 4, aggregation of the magnetic particles was observed in the sample treated with the second treatment reagent A (not containing mercaptoethylamine) in the second treatment step. In contrast, aggregation of the magnetic particles was not observed in the sample treated with the second treatment reagent B or C containing mercaptoethylamine. This shows that the addition of a reducing agent to the second treatment reagent can inhibit aggregation of the magnetic particles after the separating step.

### Example 6

### <Determining whether inhibition of magnetic particle aggregation depends on the reducing agent type and the presence of HCV>

The second treatment reagents D, E, and F shown below were prepared for the purpose of determining whether the aggregation inhibition by the reducing agent-containing second treatment reagent depends on the reducing agent type. In this example, HCV-positive serum was used in place of HCV-negative serum for the purpose of determining whether the presence or absence of HCV in serum used as the sample has an influence on the aggregation.

### Second treatment reagent

The second treatment reagents D, E, and F shown below were prepared, which were each an aqueous solution containing 0.15 M of citric acid as an acid material.

| Second treatment reagent D | | |
|---|---|---|
| | Citric acid | 0.15 M |
| | Mercaptoethylamine | 45 mM |

| Second treatment reagent E | | |
|---|---|---|
| | Citric acid | 0.15 M |
| | Dithiothreitol | 45 mM |

| Second treatment reagent F | | |
|---|---|---|
| | Citric acid | 0.15 M |
| | Cysteine hydrochloride | 90 mM |

The second treatment reagent D contains mercaptoethylamine as a reducing agent. The second treatment reagent E contains dithiothreitol as a reducing agent. The second treatment reagent F contains cysteine hydrochloride as a reducing agent.

The magnetic particle aggregation-inhibiting effect was determined as in Example 5, except that the second treatment reagents D, E, and F and HCV-positive serum were used. The second treatment reagent A was also used as a control in the determination of the magnetic particle aggregation-inhibiting effect. The results of evaluation of the aggregation are shown in Table 5.

**Table 5**

| **Second treatment reagent** | **Evaluation of particle aggregation** |
|---|---|
| A | ++ |
| D | - |
| E | - |
| F | - |

As shown in Table 5, aggregation of magnetic particles was observed in the sample treated with the second treatment reagent A (not containing mercaptoethylamine) in the second treatment step even when HCV-positive serum was used. In contrast, aggregation of magnetic particles was not observed in the sample treated with the second treatment reagent D containing mercaptoethylamine as in Example 1 even when HCV-positive serum was used. Aggregation of magnetic particles was also not observed in the samples treated with the second treatment reagent E containing dithiothreitol and the second treatment reagent F containing cysteine hydrochloride as in the case of the second treatment reagent D. This shows that aggregation of magnetic particles after the separating step can be inhibited by adding any type of reducing agent to the second treatment reagent. It is also shown that regardless of the presence or absence of HCV in serum, aggregation of magnetic particles after the separating step can be inhibited by adding a reducing agent to the second treatment reagent.

### Example 7

### <Determining whether an inorganic salt inhibits aggregation of magnetic particles>

The second treatment reagents G, H, and I shown below were prepared for the purpose of determining whether the aggregation inhibition by the second treatment reagent containing a reducing agent and an inorganic salt depends on the inorganic salt type.

### Second treatment reagent

The second treatment reagents G, H, and I shown below were prepared, which were each an aqueous solution containing 0.15 M of citric acid as an acid material.

| Second treatment reagent G | | |
|---|---|---|
| | Citric acid | 0.15 M |
| | Sodium chloride | 0.6 M |

| Second treatment reagent H | | |
|---|---|---|
| | Citric acid | 0.15 M |
| | Potassium chloride | 0.6 M |

| Second treatment reagent I | | |
|---|---|---|
| | Citric acid | 0.15 M |
| | Sodium sulfate | 0.6 M |

The second treatment reagent G contains sodium chloride as an inorganic salt. The second treatment reagent H contains potassium chloride as an inorganic salt. The second treatment reagent I contains sodium sulfate as an inorganic salt.

The magnetic particle aggregation-inhibiting effect was determined as in Example 6, except that the second treatment reagents G, H, and I were used. The second treatment reagent A was also used as a control in the determination of the magnetic particle aggregation-inhibiting effect. The results of evaluation of the aggregation are shown in Table 6.

**Table 6**

| Second treatment reagent | Evaluation of particle aggregation |
|---|---|
| A | ++ |
| G | + |
| H | + |
| I | + |

As shown in Table 6, aggregation of magnetic particles was observed in the sample treated with the second treatment reagent A (containing no inorganic salt) in the second treatment step. In contrast, the second treatment reagents G, H, and I containing sodium chloride, potassium chloride, and sodium sulfate, respectively, were found to be effective in inhibiting aggregation of magnetic particles as compared with the second treatment reagent A, although magnetic particle aggregation was slightly observed in the samples treated with these reagents. This shows that inorganic salts are also effective in inhibiting aggregation of magnetic particles, although the magnetic particle aggregation-inhibiting effect of inorganic salts is lower than that of reducing agents. It is therefore suggested that aggregation of magnetic particles can be more effectively inhibited by adding a reducing agent and an inorganic salt.

### Example 8

### <Determination of the presence or absence of HCV by immune complex transfer assay using a reducing agent-containing pretreatment liquid>

The experiment described below was performed using HCV-positive serum Nos. 1 to 42 obtained from HCV-positive patients.

The reagents used in this example are shown below.

### First treatment reagent

The same reagent as in Example 5 was used.

### Second treatment reagent

The same reagent as the second treatment reagent C in Example 5 was used.

### Labeled antibody reagent

Ten µl of the ALP-labeled HCF3-807 prepared using the same process as in Example 5 was diluted 60-fold with 590 µl of a labeled antibody diluent (25 mM Tris-HCl (pH 7.5), 0.15 M NaCl, 0.25% BSA, 0.02% NaN₃, 1 mM MgCl₂, 0.1 mM ZnCl₂) to form a labeled antibody reagent.

### First antibody reagent

Five µl of the biotin/DNP-labeled HCF4-104 prepared using the same process as in Example 5 was diluted 100-fold with 495 µl of a diluent (25 mM Tris-HCl (pH 7.5), 0.15 M NaCl, 0.25% BSA, 0.02% NaN₃) to form a first antibody reagent.

### Magnetic particle reagent

An anti-DNP antibody (DNP-1753) prepared using the same process as in Example 5 was immobilized on the magnetic particles to be used.

### Solid phase

The solid phase used was a streptavidin-immobilized plate (streptavidin plate). The streptavidin plate was prepared by allowing 100 µl of a solution of 10 µg/ml streptavidin (Wako Pure Chemical Industries, Ltd.) to act on C8 WHITE MAXISORP Plate (manufactured by NUNC A/S.) at room temperature.

### Dissociating agent

DNP-Lys was used as a material for dissociating the binding between the first and second antibodies. DNP-Lys (manufactured by Tokyo Chemical Industry Co., Ltd.) was diluted to 0.2 mM with a diluent (25 mM Tris-HCl (pH 7.5), 0.6 M NaCl, 0.25% BSA, 0.02% NaN₃) to form a dissociating agent.

The presence or absence of an HCV core protein in HCV-positive serum Nos. 1 to 42 was determined as described below.

### <First treatment step>

A mixture of 40 µl of HCV-positive serum and 60 µl of the first treatment reagent was incubated at room temperature for 8 minutes, so that the first treatment step was completed.

### <Second treatment step>

Sixty µl of the second treatment reagent was added to the sample obtained in the first treatment step and then incubated at room temperature for 5 minutes, so that the second treatment step was completed. After the second treatment step, it was confirmed that the sample had a pH of 7 to 7.5 using pH test paper (manufactured by Whatman plc.).

### <Forming step>

Seventy five µl of the labeled antibody reagent and 160 µl of the sample obtained in the second treatment step were mixed in a reaction cuvette (reaction cuvette for HISCL, manufactured by SYSMEX CORPORATION) and incubated at room temperature for 10 minutes. Subsequently, 50 µl of the first antibody reagent was added to the reaction cuvette and incubated at room temperature for 10 minutes. Subsequently, 80 µl of the magnetic particle reagent was added to the reaction cuvette and incubated at room temperature for 10 minutes, so that the forming step was completed.

### <Separating step>

The reaction cuvette containing the sample obtained in the forming step was subjected to magnetic separation using a magnetic separator (MiniTube Mag Separator manufactured by Spherotech, Inc.). The supernatant was removed, 380 µl of a washing beffer (HISCL washing buffer manufactured by SYSMEX CORPORATION) was added to the reaction cuvette, and washing accompanied by magnetic separation was performed again. After the washing was repeated twice, washing with 190 µl of the washing buffer was performed once, so that the separating step was completed.

### <Transferring step>

Forty µl of the dissociating agent was added to the reaction cuvette containing the sample obtained in the separating step and incubated at room temperature for 4 minutes. The sample was then subjected to magnetic separation, and the supernatant was transferred to the solid phase and incubated at room temperature for 20 minutes. Subsequently, the supernatant was removed, 300 µl of the washing buffer was added to the solid phase, and washing accompanied by removal of the supernatant was performed again. The washing was repeated five times, so that the transferring step was completed.

### <Measuring step>

Twenty µl of HISCL R4 reagent (manufactured by SYSMEX CORPORATION) and 20 µl of HISCL R5 reagent were added to the solid phase containing the sample obtained in the transferring step and incubated at 42°C for 4 minutes. The luminescence intensity (counts) was measured by photometry (3 seconds at gain 4095) using FLUOstar OPTIMA (manufactured by BMG LABTECH GmbH), so that the measuring step was completed.

### <Determining step>

The presence or absence of an HCV core protein in the sample was determined based on the value (measured value) of the luminescence intensity obtained in the measuring step. Specifically, when the measured value of each sample was equal to or more than the threshold value, it was determined that HCV was present in the sample, and when the measured value was less than the threshold value, it was determined that HCV was absent in the sample. The process from the first treatment step to the measuring step was performed on 103 HCV-negative serum samples, and the average of the resulting measured values was calculated. The threshold value was defined as the value obtained by multiplying the standard deviation of the average by 15 and adding the resulting product to the average. In this experiment, the average of the measured values was 2054.6, the standard deviation was 125.3, and the threshold value was 3934.4.

### Comparative Example 1

### <Determination of the presence or absence of HCV by a conventional technique>

The presence or absence of HCV was determined by a conventional technique using Lumispot Eiken HCV Antigen (registered trademark, manufactured by Eiken Chemical Co., Ltd.). The HCV-positive serum Nos. 1 to 42 were treated according to the protocol described in the instructions attached to Lumispot Eiken HCV Core Antigen, and the presence or absence of HCV was determined by detecting HCV antigen in each HCV-positive serum sample using a full automatic chemiluminescence enzyme immunoassay system LS-2000 (registered trademark, manufactured by Eiken Chemical Co., Ltd.). The limit of HCV antigen detection with Lumispot Eiken HCV Antigen, namely, the threshold for determining the presence or absence of HCV is 0.4 pg/ml.

Table 7 shows the measurement results and the determination results in Comparative Example 1 and Example 8 using HCV-positive serum Nos. 1 to 42.

The symbols used in Table 7 represent as follows. O: HCV was determined to be present in the sample. X: HCV was determined to be absent in the sample. **-:** Less than the detection limit.

**Table 7**

| **HCV- positive serum No.** | **Measurement results in Comparative Example 1(pg/ml)** | **Determination results in Comparative Example 1** | **Measurement results in Example 8(Counts)** | **Determination results in Example 8** |
|---|---|---|---|---|
| 1 | 1.82 | ○ | 697451 | ○ |
| 2 | - | × | 27100 | ○ |
| 3 | - | × | 75126 | ○ |
| 4 | 0.5 | ○ | 164279 | ○ |
| 5 | 346 | ○ | 560988 | ○ |
| 6 | 7.52 | ○ | 759943 | ○ |
| 7 | - | × | 3406 | × |
| 8 | - | × | 16122 | ○ |
| 9 | 2 42 | ○ | 366948 | ○ |
| 10 | - | × | 12340 | ○ |
| 11 | - | × | 2263 | × |
| 12 | - | × | 2227 | × |
| 13 | - | × | 2041 | × |
| 14 | - | × | 1862 | × |
| 15 | - | × | 61866 | ○ |
| 16 | - | × | 2456 | × |
| 17 | 0.44 | ○ | 9750 | ○ |
| 18 | 4.08 | ○ | 359053 | ○ |
| 19 | 0.78 | ○ | 12377 | ○ |
| 20 | 1.84 | ○ | 146267 | ○ |
| 21 | 9.48 | ○ | 1211240 | ○ |
| 22 | 1.18 | ○ | 15517 | ○ |
| 23 | 2.68 | ○ | 17600 | ○ |
| 24 | 2.68 | ○ | 92208 | ○ |
| 25 | 5.06 | ○ | 530672 | ○ |
| 26 | 16.72 | ○ | 784410 | ○ |
| 27 | 23.4 | ○ | 130081 | ○ |
| 28 | 33.64 | ○ | 1769110 | ○ |
| 29 | 50 | ○ | 2371871 | ○ |
| 30 | 130.4 | ○ | 374083 | ○ |
| 31 | 68.4 | ○ | 2580294 | ○ |
| 32 | 72.8 | ○ | 4036040 | ○ |
| 33 | 84 | ○ | 1854891 | ○ |
| 34 | 76.4 | ○ | 4935006 | ○ |
| 35 | 98.4 | ○ | 6392310 | ○ |
| 36 | 106.4 | ○ | 6200307 | ○ |
| 37 | 145.6 | ○ | 5655908 | ○ |
| 38 | 206 | ○ | 10260373 | ○ |
| 39 | 340 | ○ | 21178181 | ○ |
| 40 | 283.6 | ○ | 2453071 | ○ |
| 41 | 270.4 | ○ | 2775536 | ○ |
| 42 | 352.8 | ○ | 33754029 | ○ |

Table 7 shows that in Comparative Example 1, the presence of HCV was not able to be determined for 11 of the HCV-positive serum samples (HCV-positive serum Nos. 2, 3, 7, 8, and 10 to 16). In Example 8, however, the presence of HCV in the sample was able to be determined for 5 of the 11 samples (HCV-positive serum Nos. 2, 3, 8, 10, and 15). In Example 8, the presence of HCV was also able to be determined for all of the 31 samples for which the presence of HCV was able to be determined in Comparative Example 1. Therefore, it was demonstrated that the presence or absence of HCV was determined more accurately in Example 8 than in Comparative Example 1. This suggests that the presence or absence of hepatitis C virus in a sample can be more accurately determined by the immune complex transfer assay using a reducing agent-containing pretreatment liquid.

## Claims

1. A method for detecting a hepatitis C virus (HCV) core protein in a sample by an immune complex transfer assay using particles as a solid phase, comprising:
treating the sample suspected of containing hepatitis C virus with an alkaline material-containing reagent, which contains an alkaline material, a nonionic surfactant and a chaotropic agent;
treating the sample obtained in the first treating step with an acid material-containing reagent which contains an acid material and a reducing agent, wherein the reducing agent is at least one selected from mercaptoethylamine, mercaptoethanol, dithiothreitol, cysteine, dithioerythritol, sodium borohydride, and phosphine, and
detecting said HCV core protein in said sample obtained in the second step with an immune complex transfer assay using particles as a solid phase.

2. The method according to claim 1, wherein the nonionic surfactant is selected from polyoxyethylene nonionic surfactants.

3. The method according to claim 1 or 2, wherein the chaotropic agent is at least one selected from urea, guanidine hydrochloride, sodium salicylate, acetamide, and formaldehyde.

4. The method according to any one of claims 1 to 3, wherein the alkaline material is at least one selected from sodium hydroxide, potassium hydroxide, and magnesium hydroxide.

5. The method according to any one of claims 1 to 4, wherein the acid material is at least one selected from acetic acid, lactic acid, citric acid, malic acid, succinic acid, phosphoric acid, formic acid, fumaric acid, tartaric acid, hydrochloric acid, and sulfuric acid.

6. The method according to any one of claims 1 to 5, wherein the particles are magnetic particles.

7. The method according to any one of claims 1 to 6, wherein at least one of the reagents used in the first and second treating steps contains an inorganic salt.

8. The method according to claim 7, wherein the inorganic salt is at least one selected from sodium chloride, potassium chloride, magnesium chloride, and sodium sulfate.

9. The method according to claim 6, wherein the immune complex transfer assay comprises forming a complex comprising a HCV core protein, an anti-HCV core protein antibody, and magnetic particles in a liquid phase, separating the complex from the liquid phase by magnetic separation, and detecting the HCV core protein.

10. The method according to any of claims 1 to 8, wherein the immune complex transfer assay using particles as a solid phase comprises
forming a complex comprising a labeled antibody, a first antibody binding to a HCV core protein and the HCV core protein contained in the sample obtained in the second treating step on particles, the labeled antibody is capable of binding to the HCV core protein;
separating the particles in the sample from other components in the sample after the forming step;
transferring the complex formed on the particles obtained in the separating step to a solid phase other than the particles;
measuring the label of the complex obtained in the transferring step and transferred to the solid phase other than the particles; and
determining whether hepatitis C virus is present or absent in the sample, based on the result of the measuring step.

11. The method according to any one of claims 1 to 8, wherein the immune complex transfer assay using particles as a solid phase comprises:
forming, on the particles, a complex comprising a HCV core protein, a labeled antibody binding to the HCV core protein, and a first antibody binding to the HCV core protein; and
measuring the label of the complex.

12. The method according to claims 10 or 11, wherein the labeled antibody and the first antibody bind to different sites of the HCV core protein, and the sites to which they bind do not contain the 49th amino acid of the HCV core protein set forth in SEQ ID NO:2.

13. The method according to claim 12, wherein the site to which the labeled antibody or the first antibody binds is the amino acid sequence of amino acids 21 to 30 or amino acids 41 to 48 of the HCV core protein set forth in SEQ ID NO:2.

14. The method according to claim 13, wherein the labeled antibody or the first antibody is a monoclonal antibody produced by a hybridoma selected from hybridomas deposited under accession numbers NITE BP-844 and NITE BP-843 at Incorporated Administrative Agency, National Institute of Technology and Evaluation, Patent Microorganisms Depositary (NPMD).

15. A reagent kit for detection of a hepatitis C virus (HCV) core protein by an immune complex transfer assay using particles as a solid phase, comprising:
a first reagent containing an alkaline material, a nonionic surfactant and a chaotropic agent;
a second reagent containing an acid material and a reducing agent, wherein
said reducing agent is at least one selected from mercaptoethylamine, mercaptoethanol, dithiothreitol, cysteine, dithioerythritol, sodium borohydride, and phosphine;
a third reagent containing a labeled antibody capable of binding to the HCV core protein;
a fourth reagent containing a first antibody capable of binding to the HCV core protein;
a fifth reagent containing particles;
a sixth reagent containing a second antibody capable of binding to the first antibody and the particles; and
a solid phase on which a material capable of binding to the first antibody is immobilized.

16. The reagent kit according to claim 15, wherein at least one of the first and second reagents contains an inorganic salt.

17. The reagent kit according to claim 15 or 16, wherein the labeled antibody and the first antibody bind to different sites of the HCV core protein, and the sites to which they bind do not contain the 49th amino acid of the HCV core protein set forth in SEQ ID NO:2.

18. The reagent kit according to claim 17, wherein the site to which the labeled antibody or the first antibody binds is the amino acid sequence of amino acids 21 to 30 or amino acids 41 to 48 of the HCV core protein set forth in SEQ ID NO:2.

19. The reagent kit according to claim 18, wherein the labeled antibody or the first antibody is a monoclonal antibody produced by a hybridoma selected from hybridomas deposited under accession numbers NITE BP-844 and NITE BP-843 at Incorporated Administrative Agency, National Institute of Technology and Evaluation, Patent Microorganisms Depositary (NPMD).

## Patentansprüche

1. Verfahren zum Nachweisen eines Kernproteins des Hepatitis-C-Virus (HCV) in einer Probe mit einem Immunkomplextransferassay unter Verwendung von Partikeln als Festphase, umfassend:
Behandeln der Probe, bei der vermutet wird, dass sie Hepatitis-C-Virus enthält, mit einem alkalisches Material enthaltenden Reagens, das ein alkalisches Material, ein nicht-ionisches Tensid und ein chaotropes Mittel enthält;
Behandeln der in dem ersten Behandlungsschritt erhaltenen Probe mit einem saures Material enthaltenden Reagens, das ein saures Material und ein Reduktionsmittel enthält, wobei es sich bei dem Reduktionsmittel um mindestens eines handelt, das aus Mercaptoethylamin, Mercaptoethanol, Dithiothreitol, Cystein, Dithioerythritol, Natriumborhydrid und Phosphin ausgewählt ist, und
Nachweisen des HCV-Kernproteins in der in dem zweiten Schritt erhaltenen Probe mit einem Immunkomplextransferassay unter Verwendung von Partikeln als Festphase.

2. Verfahren nach Anspruch 1, wobei das nichtionische Tensid aus nicht-ionischen Polyoxyethylen-Tensiden ausgewählt ist.

3. Verfahren nach Anspruch 1 oder 2, wobei es sich bei dem chaotropen Mittel um mindestens eines handelt, das aus Harnstoff, Guanidinhydrochlorid, Natriumsalicylat, Acetamid und Formaldehyd ausgewählt ist.

4. Verfahren nach einem der Ansprüche 1 bis 3, wobei es sich bei dem alkalischen Material um mindestens eines handelt, das aus Natriumhydroxid, Kaliumhydroxid und Magnesiumhydroxid ausgewählt ist.

5. Verfahren nach einem der Ansprüche 1 bis 4, wobei es sich bei dem sauren Material um mindestens eines handelt, das aus Essigsäure, Milchsäure, Zitronensäure, Äpfelsäure, Bernsteinsäure, Phosphorsäure, Ameisensäure, Fumarsäure, Weinsäure, Salzsäure und Schwefelsäure ausgewählt ist.

6. Verfahren nach einem der Ansprüche 1 bis 5, wobei es sich bei den Partikeln um magnetische Partikel handelt.

7. Verfahren nach einem der Ansprüche 1 bis 6, wobei mindestens eines der in dem ersten und zweiten Behandlungsschritt verwendeten Reagenzien ein anorganisches Salz enthält.

8. Verfahren nach Anspruch 7, wobei es sich bei dem anorganischen Salz um mindestens eines handelt, das aus Natriumchlorid, Kaliumchlorid, Magnesiumchlorid und Natriumsulfat ausgewählt ist.

9. Verfahren nach Anspruch 6, wobei der Immunkomplextransferassay das Bilden eines Komplexes, der ein HCV-Kernprotein, einen Anti-HCV-Kernprotein-Antikörper und magnetische Partikel umfasst, in einer Flüssigphase, Trennen des Komplexes von der Flüssigphase durch magnetische Trennung und Nachweisen des HCV-Kernproteins umfasst.

10. Verfahren nach einem der Ansprüche 1 bis 8, wobei der Immunkomplextransferassay unter Verwendung von Partikeln als Festphase Folgendes umfasst:
Bilden eines Komplexes, welcher einen markierten Antikörper, einen ersten, an ein HCV-Kernprotein bindenden Antikörper und das in der im zweiten Behandlungsschritt erhaltenen HCV-Kernprotein umfasst, auf Partikeln, wobei der markierte Antikörper zum Binden an das HCV-Kernprotein in der Lage ist;
Trennen der Partikel in der Probe von anderen Bestandteilen in der Probe nach dem Bildungsschritt;
Übertragen des auf den in dem Trennschritt erhaltenen Partikeln gebildeten Komplexes an eine andere Festphase als den Partikeln;
Messen der Markierung des im Übertragungsschritt erhaltenen und auf die andere Festphase als die Partikel übertragenen Komplexes; und
Nachweisen, ob Hepatitis-C-Virus in der Probe vorhanden oder nicht vorhanden ist, ausgehend von dem Ergebnis des Messschrittes.

11. Verfahren nach einem der Ansprüche 1 bis 8, wobei der Immunkomplextransferassay unter Verwendung von Partikeln als Festphase Folgendes umfasst:
Bilden eines Komplexes, welcher ein HCV-Kernprotein, einen an das HCV-Kernprotein bindenden, markierten Antikörper und einen ersten, an das HCV-Kernprotein bindenden Antikörper umfasst, auf den Partikeln, und
Messen der Markierung des Komplexes.

12. Verfahren nach Anspruch 10 oder 11, wobei der markierte Antikörper und der erste Antikörper an verschiedene Stellen des HCV-Kernproteins binden und die Stellen, an die sie binden, nicht die 49ste Aminosäure des in SEQ ID Nr. 2 ausgeführten HCV-Kernproteins enthalten.

13. Verfahren nach Anspruch 12, wobei es sich bei der Stelle, an die der markierte Antikörper und der erste Antikörper binden, um die Aminosäuresequenz von Aminosäure 21 bis 30 oder Aminosäure 41 bis 48 des in SEQ ID Nr. 2 ausgeführten HCV-Kernproteins handelt.

14. Verfahren nach Anspruch 13, wobei es sich bei dem markierten Antikörper oder dem ersten Antikörper um einen monoklonalen Antikörper handelt, der von einem Hybridom gebildet wird, das aus Hybridomen ausgewählt ist, welche unter Zugangsnummer NITE BP-844 und NITE BP-843 bei der Incorporated Administrative Agency, National Institute of Technologie and Evaluation, Patent Microorganisms Depositary (NPMD) hinterlegt sind.

15. Reagenskit zum Nachweisen eines Kernproteins des Hepatitis-C-Virus (HCV) mit einem Immunkomplextransferassay unter Verwendung von Partikeln als Festphase, umfassend:
ein erstes Reagens, das ein alkalisches Material, ein nicht-ionisches Tensid und ein chaotropes Mittel enthält;
ein zweites Reagens, das ein saures Material und ein Reduktionsmittel enthält, wobei
es sich bei dem Reduktionsmittel um mindestens eines handelt, das aus Mercaptoethylamin, Mercaptoethanol, Dithiothreitol, Cystein, Dithioerythritol, Natriumborhydrid und Phosphin ausgewählt ist;
ein drittes Reagens, das einen markierten Antikörper enthält, der zum Binden an das HCV-Kernprotein in der Lage ist;
ein viertes Reagens, das einen ersten Antikörper enthält, der zum Binden an das HCV-Kernprotein in der Lage ist;
ein fünftes, Partikel enthaltendes Reagens;
ein sechstes Reagens, das einen zweiten Antikörper enthält, der zum Binden an den ersten Antikörper und die Partikel in der Lage ist; und
eine Festphase, auf welcher ein Material, das zum Binden des ersten Antikörpers in der Lage ist, immobilisiert ist.

16. Reagenskit nach Anspruch 15, wobei mindestens eines der ersten und zweiten Reagenzien ein anorganisches Salz enthält.

17. Reagenskit nach Anspruch 15 oder 16, wobei der markierte Antikörper und der erste Antikörper an verschiedene Stellen des HCV-Kernproteins binden und die Stellen, an die sie binden, nicht die 49ste Aminosäure des in SEQ ID Nr. 2 ausgeführten HCV-Kernproteins enthalten.

18. Reagenskit nach Anspruch 17, wobei es sich bei der Stelle, an die der markierte Antikörper und der erste Antikörper binden, um die Aminosäuresequenz von Aminosäure 21 bis 30 oder Aminosäure 41 bis 48 des in SEQ ID Nr. 2 ausgeführten HCV-Kernproteins handelt.

19. Reagenskit nach Anspruch 18, wobei es sich bei dem markierten Antikörper oder dem ersten Antikörper um einen monoklonalen Antikörper handelt, der von einem Hybridom gebildet wird, das aus Hybridomen ausgewählt ist, welche unter Zugangsnummer NITE BP-844 und NITE BP-843 bei der Incorporated Administrative Agency, National Institute of Technologie and Evaluation, Patent Microorganisms Depositary (NPMD) hinterlegt sind.

## Revendications

1. Dispositif de détection de la protéine core du virus de l'hépatite C (VHC) dans un échantillon à l'aide d'une technique de transfert de complexe immunologique utilisant des particules comme phase solide, comprenant :
le traitement de l'échantillon suspecté de contenir le virus de l'hépatite C à l'aide d'un réactif alcalin, contenant un produit alcalin, un surfactant non ionique et un agent chaotropique ;
le traitement de l'échantillon obtenu lors de la première phase de traitement à l'aide d'un réactif acide, contenant un produit acide et un agent de réduction, dans lequel l'agent de réduction est au moins l'un des produits sélectionnés parmi la mercaptoéthylamine, le mercaptoéthanol, le dithiothréitol, la cystéine, le dithioérythritol, le borohydrure de sodium et la phosphine, et
la détection de la protéine core du VHC dans l'échantillon obtenu lors de la seconde phase de traitement à l'aide d'une technique de transfert de complexe immunologique utilisant des particules comme phase solide.

2. Dispositif selon la revendication 1, dans lequel le surfactant non ionique est sélectionné parmi les surfactants non ioniques de type polyoxyéthylène.

3. Dispositif selon les revendications 1 ou 2, dans lequel l'agent chaotropique est au moins l'un des produits sélectionnés parmi l'urée, l'hydrochlorure de guanidine, le salicylate de sodium, l'acétamide et le formaldéhyde.

4. Dispositif selon l'une quelconque des revendications 1 à 3, dans lequel le produit alcalin est au moins l'un des produits sélectionnés parmi l'hydroxyde de sodium, l'hydroxyde de potassium et l'hydroxyde de magnésium.

5. Dispositif selon l'une quelconque des revendications 1 à 4, dans lequel le produit acide est au moins l'un des produits sélectionnés parmi l'acide acétique, l'acide lactique, l'acide citrique, l'acide malique, l'acide succinique, l'acide phosphorique, l'acide formique, l'acide fumarique, l'acide tartrique, l'acide chlorhydrique et l'acide sulfurique.

6. Dispositif selon l'une quelconque des revendications 1 à 5, dans lequel les particules sont des particules magnétiques.

7. Dispositif selon l'une quelconque des revendications 1 à 6, dans lequel au moins un des réactifs utilisés pour la première et la seconde phases de traitement contient un sel inorganique.

8. Dispositif selon la revendication 7, dans lequel le sel inorganique est au moins l'un des produits sélectionnés parmi le chlorure de sodium, le chlorure de potassium, le chlorure de magnésium et le sulfate de sodium.

9. Dispositif selon la revendication 6, dans lequel la technique de transfert de complexe immunologique consiste en la formation d'un complexe comprenant la protéine core du VHC, un anticorps anti-protéine core du VHC et des particules magnétiques en phase liquide, la séparation du complexe de la phase liquide par séparation magnétique et la détection de la protéine core du VHC.

10. Dispositif selon l'une quelconque des revendications 1 à 8, dans lequel la technique de transfert de complexe immunologique utilisant des particules comme phase solide comprend :
la formation d'un complexe comprenant un anticorps marqué, un premier anticorps se liant à une protéine core du VHC et la protéine core du VHC contenue dans l'échantillon obtenu lors de la seconde phase de traitement sur les particules, l'anticorps marqué étant capable de se lier à la protéine core du VHC ;
la séparation des particules de l'échantillon des autres composants dans l'échantillon après l'étape de formation ;
le transfert du complexe formé sur les particules obtenu lors de l'étape de séparation à une phase solide autre que les particules;
la mesure du marqueur du complexe obtenu lors du transfert et transféré à la phase solide autre que les particules ; et
la détermination de la présence ou de l'absence du virus de l'hépatite C dans l'échantillon, en fonction des résultats de l'étape de mesure.

11. Dispositif selon l'une quelconque des revendications 1 à 8, dans lequel la technique de transfert de complexe immunologique utilisant des particules comme phase solide comprend :
la formation, sur les particules, d'un complexe comprenant une protéine core du VHC, un anticorps marqué se liant à la protéine core du VHC et un premier anticorps se liant à la protéine core du VHC ; et
la mesure du marqueur du complexe.

12. Dispositif selon les revendications 10 ou 11, dans lequel l'anticorps marqué et le premier anticorps se lient à différents sites de la protéine core du VHC, et les sites auxquels ils se lient ne contiennent pas le 49ème acide aminé de la protéine core du VHC situé dans la SEQ ID n°2.

13. Dispositif selon la revendication 12, dans lequel le site auquel l'anticorps marqué ou le premier anticorps se lient est la séquence d'acides aminés 21 à 30 ou 41 à 48 de la protéine core du VHC située dans la SEQ ID n°2.

14. Dispositif selon la revendication 13, dans lequel l'anticorps marqué ou le premier anticorps est un anticorps monoclonal produit par un hybridome sélectionné parmi des hybridomes déposés sous les numéros d'accès NITE BP-844 et NITE BP-843 auprès de l'organisme administratif constitué en société, National Institute of Technology and Evaluation, Patent Microorganisms Depositary (NPMD).

15. Kit de réactifs pour la détection de la protéine core du virus de l'hépatite C (VHC) par une technique de transfert de complexe immunologique utilisant des particules comme phase solide, comprenant :
un premier réactif comprenant un produit alcalin, un surfactant non ionique et un agent chaotropique ;
un second réactif comprenant un produit acide et un agent de réduction, dans lequel l'agent de réduction est au moins l'un des produits sélectionnés parmi la mercaptoéthylamine, le mercaptoéthanol, le dithiothréitol, la cystéine, le dithioérythritol, le borohydrure de sodium et la phosphine ;
un troisième réactif comprenant un anticorps marqué capable de se lier à la protéine core du VHC ;
un quatrième réactif comprenant un premier anticorps capable de se lier à la protéine core du VHC ;
un cinquième réactif comprenant des particules ;
un sixième réactif comprenant un second anticorps capable de se lier au premier anticorps et aux particules ; et
une phase solide sur laquelle un produit capable de se lier au premier anticorps est immobilisé.

16. Kit de réactifs selon la revendication 15, dans lequel au moins un du premier et du second réactifs contient un sel inorganique.

17. Kit de réactifs selon les revendications 15 ou 16, dans lequel l'anticorps marqué et le premier anticorps se lient à différents sites de la protéine core du VHC, et les sites auxquels ils se lient ne contiennent pas le 49ème acide aminé de la protéine core du VHC situé dans la SEQ ID n°2.

18. Kit de réactifs selon la revendication 17, dans lequel le site auquel l'anticorps marqué ou le premier anticorps se lient est la séquence d'acides aminés 21 à 30 ou 41 à 48 de la protéine core du VHC située dans la SEQ ID n°2.

19. Kit de réactifs selon la revendication 18, dans lequel l'anticorps marqué ou le premier anticorps est un anticorps monoclonal produit par un hybridome sélectionné parmi des hybridomes déposés sous les numéros d'accès NITE BP-844 et NITE BP-843 auprès de l'organisme administratif constitué en société, National Institute of Technology and Evaluation, Patent Microorganisms Depositary (NPMD).
